# EUROPEAN PATENT APPLICATION

(11) **EP 4 793 354 A2**
(43) Date of publication of application: **19.08.2026**
(21) Application number: 26153950.6
(22) Date of filing: 23.01.2026
(51) Int. Cl.: C12N 15/11, C12N 15/113

(54) **MISMATCHED SIRNA, SIRNA CONJUGATE, PHARMACEUTICAL COMPOSITION AND USE THEREOF**

(30) Priority: 23.01.2025 CN 202510106825
(71) Applicant: Chengdu Gowell Biopharmaceutical Co., Ltd., Chengdu, Sichuan 610093 (CN)
(72) Inventor: HUANG, Yi, Chengdu 610093 (CN); WEI, Chuanlai, Chengdu 610093 (CN); ZHOU, Wei, Chengdu 610093 (CN); CHEN, Youjin, Chengdu 610093 (CN); WU, Yueqi, Chengdu 610093 (CN); LV, Feilong, Chengdu 610093 (CN)
(74) Representative: Bayramoglu et al.

(57) **Abstract**

The present invention provides a mismatched siRNA for inhibiting gene expression, an siRNA conjugate, a pharmaceutical composition, and uses thereof, the siRNA comprises a sense strand and an antisense strand; wherein the sense strand is 17-21 continuous nucleotides in length and the antisense strand is 19-23 continuous nucleotides in length; the sense strand and the antisense strand comprise one or more of a 2'-methoxy modified nucleotide, a 2'-fluoro modified nucleotide, and a 5'-VP modified nucleotide, and comprise one or more phosphorothioate groups located between two adjacent nucleotides; and at least one nucleotide at a position greater than 15 of the antisense strand in the 5' end to 3' end direction is mismatched to the target gene.

## Description

### CROSS REFERENCE TO THE RELATED APPLICATIONS

This application is based upon and claims priority to Chinese Patent Application No. 202510106825.X, filed on January 23, 2025, the entire contents of which are incorporated herein by reference.

### TECHNICAL FIELD

The present disclosure relates to the field of medical technology and particularly relates to a mismatched siRNA for inhibiting expression of a target gene, a siRNA conjugate, a pharmaceutical composition, and uses thereof.

### BACKGROUND TECHNOLOGY

Small interfering RNA (siRNA) is a new and potential candidate research object, which can inhibit or block the expression of the target gene of interest in a sequence-specific manner based on the interference mechanism of RNA, so as to achieve the purpose of treating diseases. In particular, siRNA inhibits or blocks target gene expression by loading into an RNA-induced silencing complex (RISC), the guide strand of which (also referred to as the antisense strand) is complementary paired with a target nucleic acid in the mRNA of the target gene, causing degradation of the mRNA of the target gene. siRNA can specifically recognize target RNA through complementary base pairing, so as to achieve target RNA cleavage, down-regulate the level of target RNA, and inhibit the expression of the target gene. Therefore, siRNA has a very broad application prospect. The rate of progress of siRNA is striking relative to the development of traditional drug candidates, such as small-molecule compounds, as well as proteins and antibodies. Although siRNA is of unlimited value in theory, it is still unsatisfactory to translate its powerful gene silencing capacity into an applied tool, mainly for disease treatment.

### CONTENT OF THE INVENTION

Based on the above status and needs, the present disclosure provides a mismatched siRNA inhibiting the target gene expression, a siRNA conjugate, a pharmaceutical composition comprising the siRNA or the siRNA conjugate as an active ingredient, and a use of the siRNA or the siRNA conjugate or the pharmaceutical composition in the preparation of a drug for the prevention and/or treatment of a disease.

Accordingly, in a first aspect, the present disclosure provides a siRNA for inhibiting the target gene expression, the siRNA comprises a sense strand and an antisense strand, at least one nucleotide at a position greater than 15 of the antisense strand in the 5' end to 3' end direction is mismatched to the target gene.

In a second aspect, the present disclosure provides a siRNA conjugate prepared by conjugating a siRNA as described in the first aspect with a conjugating molecule.

In a third aspect, the present disclosure provides a pharmaceutical composition comprising a siRNA according to any one of the embodiments of the first aspect or a siRNA conjugate according to any one of the embodiments of the second aspect, and a pharmaceutically acceptable carrier.

In a fourth aspect, the present disclosure provides a kit comprising a siRNA according to any one of the embodiments of the first aspect and/or a siRNA conjugate according to any one of the embodiments of the second aspect and/or a pharmaceutical composition according to any one of the embodiments of the third aspect.

In a fifth aspect, the present disclosure provides a use of the siRNA according to any one of the embodiments of the first aspect and/or a siRNA conjugate according to any one of the embodiments of the second aspect and/or a pharmaceutical composition according to any one of the embodiments of the third aspect in the manufacture of a medicament for preventing and/or treating a disease.

In a sixth aspect, the present disclosure provides a method of preventing and/or treating a disease, the method includes administering a siRNA according to any one of the embodiments of the first aspect and/or a siRNA conjugate according to any one of the embodiments of the second aspect and/or a pharmaceutical composition according to any one of the embodiments of the third aspect to a subject in need thereof.

In a seventh aspect, the present disclosure provides at least one of a siRNA according to any one of the embodiments of the first aspect, a siRNA conjugate according to any one of the embodiments of the second aspect, and a pharmaceutical composition according to any one of the embodiments of the third aspect, for use in the prevention and/or treatment of a disease.

### Advantageous Effects

The siRNA, siRNA conjugates, and pharmaceutical compositions provided by the present disclosure have superior gene inhibition activity.

Additional features and advantages of the present disclosure will be set forth in the detailed description which follows.

### SPECIFIC IMPLEMENTATIONS

The following is a detailed description of specific embodiments of the present disclosure. It should be understood that the particular embodiments described herein are illustrative and explanatory only and are not restrictive of the present disclosure, but rather that various modifications, adaptations, or changes may be made based on the spirit and concepts of the present disclosure, and that such modifications, adaptations, or changes may fall within the scope of the present disclosure.

In the present disclosure, the term "siRNA" or "siRNA molecule" refers to an RNA molecule capable of sequence-specifically inducing the RNAi phenomenon, comprising a sense strand and an antisense strand, and having a partially or completely complementary double-stranded structure.

In the present disclosure, the capital letters C, G, U, A represent the base composition of ribonucleotides, unless otherwise specified; dC, dG, dT, dA represent the base composition of deoxyribonucleotides; the lowercase letter m indicates that the one nucleotide adjacent to the right side of the letter m is a 2'-methoxy modified nucleotide; the identifier i2F or lowercase letter f indicates that one nucleotide adjacent to the right side of the identifier i2F or lowercase letter f is a 2'-fluoro modified nucleotide; the identifier * indicates that the two nucleotides adjacent to the left and right of the identifier * are linked via a phosphorothioate group (respectively linked to the 5' and 3' positions of the nucleotides); the identifier # indicates a methylsulfonamidophosphonate (MsPA) linkage between two nucleotides adjacent to the left and right of the identifier #; the identifier VP indicates that one nucleotide to the right of the identifier is a vinylphosphonate-modified nucleotide, and the identifier (*E*)-VP indicates that one nucleotide to the right of the identifier (*E*)-VP is an (*E*)-vinylphosphonate-modified nucleotide (also referred to as a 5'-(*E*)-VP-modified nucleotide); an underlined " " indicates that the underlined nucleotide is a glycerol nucleic acid (GNA): 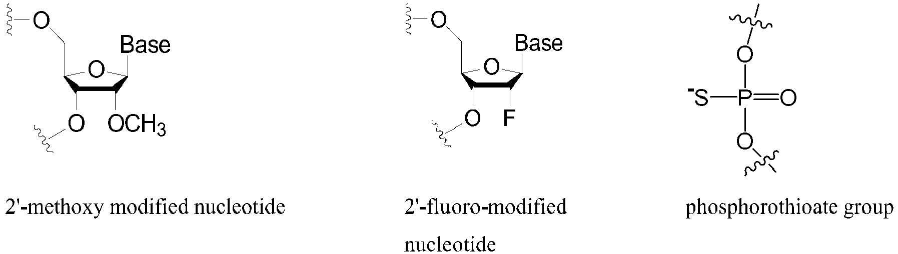 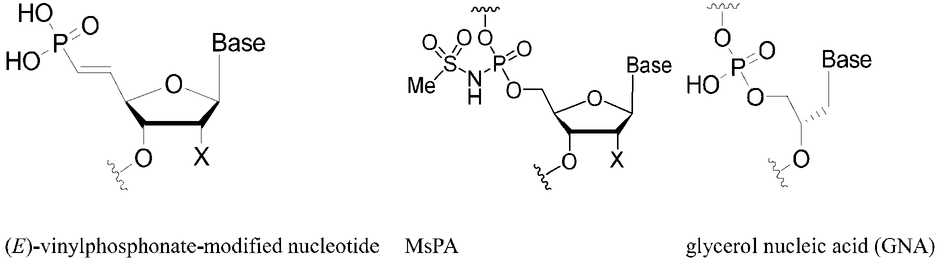 inVAb is an inverted abasic residue (also known as an "inverted abasic site" in the field), wherein invAb may have the following structures:
when located internally within the sequence, it is 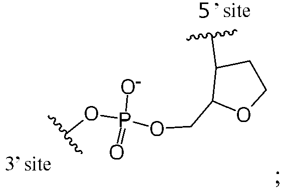
when located at the 3' end, it is 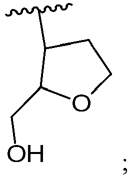 and
when located at the 5' end, it is

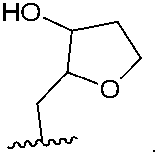

In the present disclosure, "complementary" means that, in a siRNA duplex molecule, the bases of one strand each pair with the bases of the other strand in a complementary manner, unless otherwise specified. That is, when A is paired with U and C is paired with G, the two strands are considered to be complementary. "Complementary" as described in the present disclosure, may include base-pairing formed by non-Watson-Crick base-pairing and/or non-natural or modified nucleotides, so long as they hybridize and are capable of forming a double-stranded structure. Correspondingly, in the present disclosure, "mismatched" means, unless otherwise specified, that in the siRNA duplex molecule, the bases at the corresponding positions are not paired in a complementary manner.

In the present disclosure, "substantially complementary paired" means, unless otherwise specified, that the number of mismatched nucleotides is not more than 3, for example, the number of mismatched nucleotides is 2, and the number of mismatched nucleotides is 1.

In the present disclosure, "conjugated" means that two or more chemical moieties are linked to each other by way of a covalent linkage, unless otherwise specified; a "conjugate" refers to a compound formed by covalent attachment between two or more chemical moieties; a "siRNA conjugate" refers to a compound formed by covalent attachment of one or more chemical moieties to a siRNA. It should be noted here that the various chemical moieties may be attached to the siRNA either directly or via a linker to the siRNA. For example, the conjugating molecule may comprise a linker moiety and a functional moiety (e. g., a targeting moiety for targeting to a particular tissue or cell, such as GalNAc, etc.), optionally for linking to a siRNA.

In the present disclosure, "-" in "linker-targeting ligand" means that the linker is covalently linked to the targeting ligand, unless otherwise specified.

In the present disclosure, the term "pharmaceutically acceptable" refers to carriers, vehicles, diluents, adjuvants, and/or salts/esters/hydrates thereof, and the like, that are generally chemically and physically compatible with the other ingredients including a pharmaceutical dosage form, and physiologically compatible with the recipient, unless otherwise specified.

The siRNA, siRNA conjugates described herein may assume states in which certain functional groups in their structures are protonated or deprotonated, depending on the environment, and thus, the siRNA, siRNA conjugates described herein encompass their free acid, or salt forms (including pharmaceutically acceptable salts, such as sodium, potassium, magnesium, calcium, triethylamine, spermine, or ammonium salts), with the siRNA, siRNA conjugates described herein preferably being in the sodium salt form.

In the present disclosure, the terms "2'-fluoro modified nucleotide" and "2'-fluoro nucleotide" are used interchangeably herein and refer to a nucleotide comprising a fluorine modification at the 2' position on the ribosyl group of the nucleotide, unless otherwise specified. In the present disclosure, the terms "2'-methoxy modified nucleotide" and "2'-methoxy nucleotide" are used interchangeably herein and refer to a nucleotide comprising a methoxy modification at the 2' position on the ribose group of the nucleotide, unless otherwise specified. The recitation of such 2'-fluoro nucleotides is not intended to exclude modifications at other positions of the nucleotide (e. g. other sites of the ribose group, bases, etc.), e. g. 2'-fluoro nucleotides may also comprise modifications at positions other than the 2' position of the ribose group in the nucleotide structure, and 2'-methoxy nucleotides may also comprise modifications at positions other than the 2'position in the nucleotide structure.

In the present disclosure, the term "inhibit" refers to a condition in which target gene expression is down-regulated due to siRNA-mediated mRNA degradation of the target gene, unless otherwise specified. By "down-regulation" is meant that the level of target gene expression is reduced by more than 5%, 10%, 15%, 20%, 25%, 30%, 35%, 40%, 45%, 50%, 55%, 60%, 65%, 70%, 75%, 80%, 85%, 90%, 95% or 99% or even 100% relative to the level in the absence of siRNA treatment. Where a 100% decrease in the level of target gene expression is the absence of detectable levels of target gene expression.

In this disclosure, the terms "include, comprise, and contain" or equivalents thereof are used in an open-ended fashion, and are meant to encompass not only the expressly listed elements, components, or steps, but also additional unspecified elements, components, or steps.

In the present disclosure, the term "identity" refers to similarity between two nucleotide sequences or between two amino acid sequences, unless otherwise specified. The percent identity between sequences can be determined by algorithms known to those skilled in the art (e. g., the Needleman-Wunsch algorithm, the Smith-Waterman algorithm, the BLAST algorithm). The sequence identity is independent of the presence or absence of modifications in the nucleotide sequence.

In the present disclosure, the term "substantially complementary" means, unless otherwise specified, complete complementarity between two nucleic acid sequences or at least 90% (e. g. 91%, 92%, 93%, 94%, 95%, 96%, 97%, 98%, 99%, or 100%) of the nucleotides are complementary.

The terms "optional" and "optionally" mean that the subsequently described event or circumstance may or may not occur, and that the description includes instances where the event occurs and instances where it does not. For example, "optional linker" means that a linker may not be used, or a person skilled in the art would have been able to autonomously select any one of the linkers that can satisfy the target requirements, achieve the target function, or achieve the target effect according to actual requirements in the art.

Unless otherwise indicated, all numbers expressing quantities of ingredients, physical, chemical properties, reaction conditions, and so forth used herein are to be understood as being modified in all instances by the term "about". When the term "about" is used in describing the present disclosure, the term "about" indicates that there is an error value, e. g., when used in conjunction with a percentage, the term "about" means varying within ± 5%, e. g., ± 1% or ± 0.1%, of a particular value.

The terms "subject", "patient", and "individual" are used interchangeably herein and include mammals or non-mammalian vertebrates (e. g. chickens, emu, fish), including, but not limited to, domesticated animals (e. g. cows, sheep, cats, dogs, pigs, and horses), primates (e. g. humans, non-human primates such as monkeys), rabbits, and rodents (e. g. mice, rats, guinea pigs, hamsters), preferably humans.

As used herein, the terms "treating", "treatment" or "treat" refer to alleviating or abating a disease or symptom, reducing the rate of onset or development of a disease or symptom, reducing the risk of developing a disease or symptom, delaying the development of a symptom associated with a disease or symptom, diminishing or stopping a symptom associated with a disease or symptom, causing a total or partial reversal of a disease or symptom, curing a disease or symptom, or a combination thereof.

The term "prevent" refers to: by contacting (e. g., administering) an active ingredient to a subject prior to the onset of a disease, the symptoms following the onset of the disease are alleviated as compared to the situation in the absence of the contact, which does not mean that complete inhibition of the disease is necessary.

Various aspects of the present disclosure are described in further detail below.

### I. The siRNA of the present disclosure

The present disclosure provides a siRNA for inhibiting the target gene expression. The siRNA comprises a sense strand and an antisense strand, at least one nucleotide at a position greater than 15 of the antisense strand in the 5' end to 3' end direction is mismatched to the target gene.

In some embodiments of the present disclosure, there is provided any one of the following siRNAs:
1. A siRNA for inhibiting the target gene expression, the siRNA comprises a sense strand and an antisense strand, the sense strand is 17-21 continuous nucleotides in length, the antisense strand is 19-23 continuous nucleotides in length, and at least one nucleotide at a position greater than 15 of the antisense strand in the 5' end to 3' end direction is mismatched to the target gene.
2. A siRNA for inhibiting the target gene expression, the siRNA comprises a sense strand and an antisense strand, the sense strand is 17-21 continuous nucleotides in length, the antisense strand is 19-23 continuous nucleotides in length, one nucleotide at a position greater than 15 of the antisense strand in the 5' end to 3' end direction is mismatched to the target gene.
3. A siRNA for inhibiting the target gene expression, the siRNA comprises a sense strand and an antisense strand, the sense strand is 17-21 continuous nucleotides in length, the antisense strand is 19-23 continuous nucleotides in length, two nucleotides at positions greater than 15 of the antisense strand in the 5' end to 3' end direction are mismatched to the target gene.
4. A siRNA for inhibiting the target gene expression, the siRNA comprises a sense strand and an antisense strand, the sense strand is 17-21 continuous nucleotides in length, the antisense strand is 19-23 continuous nucleotides in length, three nucleotides at positions greater than 15 of the antisense strand in the 5' end to 3' end direction are mismatched to the target gene.
5. A siRNA for inhibiting the target gene expression, the siRNA comprises a sense strand and an antisense strand, the sense strand is 17-21 continuous nucleotides in length, the antisense strand is 19-23 continuous nucleotides in length, up to 3 nucleotides at positions greater than 15 of the antisense strand in the 5' end to 3' end direction are mismatched to the target gene.
6. A siRNA for inhibiting the target gene expression, the siRNA comprises a sense strand and an antisense strand, the sense strand is 17-21 continuous nucleotides in length, the antisense strand is 19-23 continuous nucleotides in length, at least one nucleotide at a position greater than 15 of the antisense strand in the 5' end to 3' end direction is mismatched to the target gene, wherein the nucleotide of the antisense strand and the target gene may be the same at the mismatch position.
7. A siRNA for inhibiting the target gene expression, the siRNA comprises a sense strand and an antisense strand, the sense strand is 17-21 continuous nucleotides in length, the antisense strand is 19-23 continuous nucleotides in length, at least one nucleotide at a position greater than 15 of the antisense strand in the 5' end to 3' end direction is mismatched to the target gene, wherein the nucleotides of the antisense strand and the sense strand are complementary paired at mismatch position.
8. A siRNA for inhibiting the target gene expression, the siRNA comprises a sense strand and an antisense strand, the sense strand is 17-21 continuous nucleotides in length, the antisense strand is 19-23 continuous nucleotides in length, at least one nucleotide at positions 16, 17, 18, 19, optionally 20, optionally 21, optionally 22, or optionally 23, of the antisense strand in the 5' end to 3' end direction is mismatched to the target gene.
9. A siRNA for inhibiting the target gene expression, the siRNA comprises a sense strand and an antisense strand, the sense strand is 17-21 continuous nucleotides in length, the antisense strand is 19-23 continuous nucleotides in length, at least one nucleotide at positions 16, 17, 18, 19, 20, or 21 of the antisense strand in the 5' end to 3' end direction is mismatched with the target gene.
10. A siRNA for inhibiting the target gene expression, the siRNA comprises a sense strand and an antisense strand, the sense strand is 17-21 continuous nucleotides in length, the antisense strand is 19-23 continuous nucleotides in length, at least one nucleotide at positions 16, 17, 18, or 19 of the antisense strand in the 5' end to 3' end direction is mismatched with the target gene.
11. A siRNA for inhibiting the target gene expression, the siRNA comprises a sense strand and an antisense strand, the sense strand is 17-21 continuous nucleotides in length, the antisense strand is 19-23 continuous nucleotides in length, and a nucleotide at position 16 of the antisense strand in the 5' end to 3' end direction is mismatched with the target gene.
12. A siRNA for inhibiting the target gene expression, the siRNA comprises a sense strand and an antisense strand, the sense strand is 17-21 continuous nucleotides in length, the antisense strand is 19-23 continuous nucleotides in length, and a nucleotide at position 17 of the antisense strand in the 5' end to 3' end direction is mismatched with the target gene.
13. A siRNA for inhibiting the target gene expression, the siRNA comprises a sense strand and an antisense strand, the sense strand is 17-21 continuous nucleotides in length, the antisense strand is 19-23 continuous nucleotides in length, and a nucleotide at position 18 of the antisense strand in the 5' end to 3' end direction is mismatched with the target gene.
14. A siRNA for inhibiting the target gene expression, the siRNA comprises a sense strand and an antisense strand, the sense strand is 17-21 continuous nucleotides in length, the antisense strand is 19-23 continuous nucleotides in length, and at least one nucleotide at positions 19, 20, or 21 of the antisense strand in the 5' end to 3' end direction is mismatched with the target gene.
15. A siRNA for inhibiting the target gene expression, the siRNA comprises a sense strand and an antisense strand, the sense strand is 17-21 continuous nucleotides in length, the antisense strand is 19-23 continuous nucleotides in length, and a nucleotide at position 19 of the antisense strand in the 5' end to 3' end direction is mismatched with the target gene.
16. A siRNA for inhibiting the target gene expression, the siRNA comprises a sense strand and an antisense strand, the sense strand is 17-21 continuous nucleotides in length, the antisense strand is 19-23 continuous nucleotides in length, and a nucleotide at position 20 of the antisense strand in the 5' end to 3' end direction is mismatched with the target gene.
17. A siRNA for inhibiting the target gene expression, the siRNA comprises a sense strand and an antisense strand, the sense strand is 17-21 continuous nucleotides in length, the antisense strand is 19-23 continuous nucleotides in length, and a nucleotide at position 21 of the antisense strand in the 5' end to 3' end direction is mismatched with the target gene.
18. A siRNA for inhibiting the target gene expression, the siRNA comprises a sense strand and an antisense strand, the sense strand is 17-21 continuous nucleotides in length, the antisense strand is 19-23 continuous nucleotides in length, and a nucleotide at position 22 of the antisense strand in the 5' end to 3' end direction is mismatched with the target gene.
19. A siRNA for inhibiting the target gene expression, the siRNA comprises a sense strand and an antisense strand, the sense strand is 17-21 continuous nucleotides in length, the antisense strand is 19-23 continuous nucleotides in length, and a nucleotide at position 23 of the antisense strand in the 5' end to 3' end direction is mismatched with the target gene.
20. A siRNA for inhibiting the target gene expression, the siRNA comprises a sense strand and an antisense strand, the sense strand is 17 to 21 continuous nucleotides in length, the antisense strand is 19 to 23 continuous nucleotides in length, a nucleotide at position 16 of the antisense strand in the 5' end to 3' end direction is mismatched with the target gene, the nucleotide of the antisense strand is identical to the nucleotide of the target gene at the mismatch position, the nucleotide of the antisense strand and the nucleotide of the sense strand are complementary paired at the mismatch position.
21. A siRNA for inhibiting the target gene expression, the siRNA comprises a sense strand and an antisense strand, the sense strand is 17 to 21 continuous nucleotides in length, the antisense strand is 19 to 23 continuous nucleotides in length, a nucleotide at position 17 of the antisense strand in the 5' end to 3' end direction is mismatched with the target gene, the nucleotide of the antisense strand is identical to the nucleotide of the target gene at the mismatch position, the nucleotide of the antisense strand and the nucleotide of the sense strand are complementary paired at the mismatch position.
22. A siRNA for inhibiting the target gene expression, the siRNA comprises a sense strand and an antisense strand, the sense strand is 17 to 21 continuous nucleotides in length, the antisense strand is 19 to 23 continuous nucleotides in length, a nucleotide at position 18 of the antisense strand in the 5' end to 3' end direction is mismatched with the target gene, the nucleotide of the antisense strand is identical to the nucleotide of the target gene at the mismatch position the nucleotide of the antisense strand and the nucleotide of the sense strand are complementary paired at the mismatch position.
23. A siRNA for inhibiting the target gene expression, the siRNA comprises a sense strand and an antisense strand, the sense strand is 17 to 21 continuous nucleotides in length, the antisense strand is 19 to 23 continuous nucleotides in length, a nucleotide at position 19 of the antisense strand in the 5' end to 3' end direction is mismatched with the target gene, the nucleotide of the antisense strand is identical to the nucleotide of the target gene at the mismatch position, the nucleotide of the antisense strand and the nucleotide of the sense strand are complementary paired at the mismatch position.
24. A siRNA for inhibiting the target gene expression, the siRNA comprises a sense strand and an antisense strand, the sense strand is 17 to 21 continuous nucleotides in length, the antisense strand is 19 to 23 continuous nucleotides in length, a nucleotide at position 20 of the antisense strand in the 5' end to 3' end direction is mismatched with the target gene, the nucleotide of the antisense strand is identical to the nucleotide of the target gene at the mismatch position, the nucleotide of the antisense strand and the nucleotide of the sense strand are complementary paired at the mismatch position.
25. A siRNA for inhibiting the target gene expression, the siRNA comprises a sense strand and an antisense strand, the sense strand is 17 to 21 continuous nucleotides in length, the antisense strand is 19 to 23 continuous nucleotides in length, a nucleotide at position 21 of the antisense strand in the 5' end to 3' end direction is mismatched with the target gene, the nucleotide of the antisense strand is identical to the nucleotide of the target gene at the mismatch position, the nucleotide of the antisense strand and the nucleotide of the sense strand are complementary paired at the mismatch position.
26. A siRNA for inhibiting the target gene expression, the siRNA comprises a sense strand and an antisense strand, the sense strand is 17-21 continuous nucleotides in length, the antisense strand is 19-23 continuous nucleotides in length, a nucleotide at position 22 of the antisense strand in the 5' end to 3' end direction is mismatched with the target gene, the nucleotide of the antisense strand is identical to the nucleotide of the target gene at the mismatch position.
27. A siRNA for inhibiting the target gene expression, the siRNA comprises a sense strand and an antisense strand, the sense strand is 17-21 continuous nucleotides in length, the antisense strand is 19-23 continuous nucleotides in length, a nucleotide at position 23 of the antisense strand in the 5' end to 3' end direction is mismatched with the target gene, the nucleotide of the antisense strand is identical to the nucleotide of the target gene at the mismatch position
28. A siRNA for inhibiting the target gene expression, the siRNA comprises a sense strand and an antisense strand, the sense strand is 17-21 continuous nucleotides in length, the antisense strand is 19-23 continuous nucleotides in length, and the nucleotides at positions 19 and 20 of the antisense strand in the 5' end to 3' end direction are mismatched with the target gene.
29. A siRNA for inhibiting the target gene expression, the siRNA comprises a sense strand and an antisense strand, the sense strand is 17-21 continuous nucleotides in length, the antisense strand is 19-23 continuous nucleotides in length, and the nucleotides at positions 19 and 21 of the antisense strand in the 5' end to 3' end direction are mismatched with the target gene.
30. A siRNA for inhibiting the target gene expression, the siRNA comprises a sense strand and an antisense strand, the sense strand is 17-21 continuous nucleotides in length, the antisense strand is 19-23 continuous nucleotides in length, and the nucleotides at positions 20 and 21 of the antisense strand in the 5' end to 3' end direction are mismatched with the target gene.
31. A siRNA for inhibiting the target gene expression, the siRNA comprises a sense strand and an antisense strand, the sense strand is 17-21 continuous nucleotides in length, the antisense strand is 19-23 continuous nucleotides in length, and the nucleotides at positions 19, 20, and 21 of the antisense strand in the 5' end to 3' end direction are mismatched with the target gene.
32. An siRNA for inhibiting the target gene expression, the siRNA comprises a sense strand and an antisense strand, the sense strand is 17 to 21 continuous nucleotides in length, the antisense strand is 19 to 23 continuous nucleotides in length, the nucleotides at positions 19 and 20 of the antisense strand in the 5' end to 3' end direction are mismatched to the target gene, the nucleotides of the antisense strand are identical to the nucleotides of the target gene at the mismatch positions, the nucleotides of the antisense strand and the nucleotides of the sense strand are complementary paired at the mismatch positions.
33. An siRNA for inhibiting the target gene expression, the siRNA comprises a sense strand and an antisense strand, the sense strand is 17 to 21 continuous nucleotides in length, the antisense strand is 19 to 23 continuous nucleotides in length, the nucleotides at positions 19 and 21 of the antisense strand in the 5' end to 3' end direction are mismatched to the target gene, the nucleotides of the antisense strand are identical to the nucleotides of the target gene at the mismatch positions, the nucleotides of the antisense strand and the nucleotides of the sense strand are complementary paired at the mismatch positions.
34. A siRNA for inhibiting the target gene expression, the siRNA comprises a sense strand and an antisense strand, the sense strand is 17 to 21 continuous nucleotides in length, the antisense strand is 19 to 23 continuous nucleotides in length, the nucleotides at positions 20 and 21 of the antisense strand in the 5' end to 3' end direction are mismatched with the target gene, the nucleotides of the antisense strand are identical to the nucleotides of the target gene at the mismatch positions, and the nucleotides of the antisense strand and the nucleotides of the sense strand are complementary paired at the mismatch positions.
35. An siRNA for inhibiting the target gene expression, the siRNA comprises a sense strand is 17 to 21 continuous nucleotides in length and an antisense strand is 19 to 23 continuous nucleotides in length, the nucleotides at positions 19, 20, and 21 of the antisense strand in the 5' end to 3' end direction are mismatched with the target gene, the nucleotides of the antisense strand are identical to the nucleotides of the target gene at the mismatch positions, and the nucleotides of the antisense strand and the nucleotides of the sense strand are complementary paired at the mismatch positions.
36. An siRNA for inhibiting the target gene expression, the siRNA comprises a sense strand and an antisense strand, the sense strand is 17 to 21 continuous nucleotides in length, the antisense strand is 19 to 23 continuous nucleotides in length, at least one nucleotide at positions 19, 20, or 21 of the antisense strand in the 5' end to 3' end direction is mismatched with the target gene, the nucleotide of the antisense strand is identical to the nucleotide of the target gene at the mismatch position, and the nucleotide of the antisense strand and the nucleotide of the sense strand are complementary paired at the mismatch position, the antisense strand in siRNA comprises an overhang of 1-4 nucleotides in length at the 3' end.
37. An siRNA for inhibiting the target gene expression, the siRNA comprises a sense strand and an antisense strand, the sense strand is 17 to 21 continuous nucleotides in length, the antisense strand is 19 to 23 continuous nucleotides in length, at least one nucleotide at positions 19, 20, or 21 of the antisense strand in the 5' end to 3' end direction is mismatched with the target gene, the nucleotide of the antisense strand is identical to the nucleotide of the target gene at the mismatch position, and the nucleotides of the antisense strand and the nucleotides of the sense strand are complementary paired at the mismatch position, the antisense strand in siRNA comprises an overhang of 1-3 nucleotides in length at the 3' end.
38. An siRNA for inhibiting the target gene expression, the siRNA comprises a sense strand and an antisense strand, the sense strand is 17 to 21 continuous nucleotides in length, the antisense strand is 19 to 23 continuous nucleotides in length, at least one nucleotide at positions 19, 20, or 21 of the antisense strand in the 5' end to 3' end direction is mismatched with the target gene, the nucleotide of the antisense strand is identical to the nucleotide of the target gene at the mismatch position, and the nucleotide of the antisense strand and the nucleotide of the sense strand are complementary paired at the mismatch position, the antisense strand comprises an overhang of 2 nucleotides in length at the 3' end.
40. An siRNA for inhibiting the target gene expression, the siRNA comprises a sense strand and an antisense strand, the sense strand is 17 to 21 continuous nucleotides in length, the antisense strand is 19 to 23 continuous nucleotides in length, at least one nucleotide at positions 19, 20, or 21 of the antisense strand in the 5' end to 3' end direction is mismatched with the target gene, the nucleotide of the antisense strand is identical to the nucleotide of the target gene at the mismatch position, and the nucleotide of the antisense strand and the nucleotide of the sense strand are complementary paired at the mismatch position, the antisense strand comprises an overhang of 2 nucleotides in length at the 3' end, the overhang of 2 nucleotides comprises in the 5' to 3' direction: CU, GG, or GA.
41. An siRNA for inhibiting the target gene expression, the siRNA comprises a sense strand and an antisense strand, the sense strand is 17 to 21 continuous nucleotides in length, the antisense strand is 19 to 23 continuous nucleotides in length, at least one nucleotide at positions 19, 20, or 21 of the antisense strand in the 5' end to 3' end direction is mismatched with the target gene, the nucleotide of the antisense strand is identical to the nucleotide of the target gene at the mismatch position, and the nucleotide of the antisense strand and the nucleotide of the sense strand are complementary paired at the mismatch position, the antisense strand comprises an overhang of 2 nucleotides in length at the 3' end, the overhang of 2 nucleotides comprises in the 5' to 3' direction: CU, GG, or GA, the 5' end of the antisense strand in the siRNA forms a blunt end with the 3' end of the sense strand.
42. An siRNA for inhibiting the target gene expression, the siRNA comprises a sense strand and an antisense strand, the sense strand is 17 continuous nucleotides in length, at least one nucleotide at positions 19, 20, or 21 of the antisense strand in the 5' end to 3' end direction is mismatched with the target gene, the nucleotide of the antisense strand is identical to the nucleotide of the target gene at the mismatch position, and the antisense strand comprises an overhang of 4 nucleotides in length at the 3' end, the 5' end of the antisense strand in the siRNA forms a blunt end with the 3' end of the sense strand.
43. An siRNA for inhibiting the target gene expression, the siRNA comprises a sense strand and an antisense strand, the sense strand is 18 continuous nucleotides in length, at least one nucleotide at positions 19, 20, or 21 of the antisense strand in the 5' end to 3' end direction is mismatched with the target gene, the nucleotide of the antisense strand is identical to the nucleotide of the target gene at the mismatch position, and the antisense strand comprises an overhang of 3 nucleotides in length at the 3' end, the 5' end of the antisense strand in the siRNA forms a blunt end with the 3' end of the sense strand.
44. An siRNA for inhibiting the target gene expression, the siRNA comprises a sense strand and an antisense strand, the sense strand is 19 continuous nucleotides in length, at least one nucleotide at positions 19, 20, or 21 of the antisense strand in the 5' end to 3' end direction is mismatched with the target gene, the nucleotide of the antisense strand is identical to the nucleotide of the target gene at the mismatch position, and optionally the nucleotide of the antisense strand and the nucleotide of the sense strand are complementary paired at the mismatch position, the antisense strand comprises an overhang of 2 nucleotides in length at the 3' end, the overhang of 2 nucleotides comprises in the 5' to 3' direction: CU, GG, or GA, the 5' end of the antisense strand in the siRNA forms a blunt end with the 3' end of the sense strand.
45. An siRNA for inhibiting the target gene expression, the siRNA comprises a sense strand and an antisense strand, the sense strand is 20 continuous nucleotides in length, at least one nucleotide at positions 19, 20, or 21 of the antisense strand in the 5' end to 3' end direction is mismatched with the target gene, the nucleotide of the antisense strand is identical to the nucleotide of the target gene at the mismatch position, and optionally the nucleotide of the antisense strand and the nucleotide of the sense strand are complementary paired at the mismatch position, the antisense strand comprises an overhang of 2 nucleotides in length at the 3' end, the overhang of 2 nucleotides comprises in the 5' to 3' direction: CU, GG, or GA, the 5' end of the antisense strand in the siRNA forms a blunt end with the 3' end of the sense strand.
46. An siRNA for inhibiting the target gene expression, the siRNA comprises a sense strand and an antisense strand, the sense strand is 21 continuous nucleotides in length, at least one nucleotide at positions 19, 20, or 21 of the antisense strand in the 5' end to 3' end direction is mismatched with the target gene, the nucleotide of the antisense strand is identical to the nucleotide of the target gene at the mismatch position, and the nucleotide of the antisense strand and the nucleotide of the sense strand are complementary paired at the mismatch position, the antisense strand comprises an overhang of 2 nucleotides in length at the 3' end, the overhang of 2 nucleotides comprises in the 5' to 3' direction: CU, GG, or GA, the 5' end of the antisense strand in the siRNA forms a blunt end with the 3' end of the sense strand.

In some embodiments of the present disclosure, the particular mismatch patterns of the present disclosure are surprisingly able to enhance the inhibitory activity of siRNA. The present disclosure adopts the manner of nucleotide alignment of the sense strand and the antisense strand at a specific position, not only can mismatch between the antisense strand and the target gene be achieved, but the nucleotides of the sense strand and the antisense strand are still complementary paired, without affecting the thermodynamic stability of the duplex.

In some embodiments of the present disclosure, the siRNA may also contain modified nucleotides or nucleotide analogs as desired, which do not result in a significant impairment or loss of the function of the siRNA to inhibit the expression of the target gene.

Currently, there are a variety of ways available in the art for modifying siRNA, including, for example, backbone modifications (e. g. phosphate group modifications), ribose group modifications, and base modifications (Watts, J. K. G. F. Deleavey, and M. J. Damha, chemically modified siRNA: tools and applications. Drug Discov Today, 2008. 13(19-20): p. 842-55).

In some embodiments of the present disclosure, at least one nucleotide in the sense or antisense strand of the siRNA is a modified nucleotide, for example, but not limited to, a nucleotide group in which a ribose group and optionally a phosphate group are modified.

In some preferred embodiments of the present disclosure, the modified nucleotide is a nucleotide modified at the 2' position of its ribose, e. g. a nucleotide modified at the 2' position as follows: 2'-deoxy, 2'-fluoro, 2'-amino, 2'-methyl, 2'-ethyl, 2'-methyl-O-methyl, 2'-ethyl-O-methyl, 2'-O-methyl (2'-OMe), 2'-O-ethyl, 2'-O-methoxyethyl (2'-O-MOE), 2'-O-ethyl-O-methyl or 2'-O-allyl.

In some embodiments of the present disclosure, all nucleotides of the sense strand of the siRNA are modified nucleotides.

In some embodiments of the present disclosure, all of the nucleotides of the antisense strand of siRNA are modified nucleotides.

In some embodiments of the present disclosure, all of the nucleotides of the antisense strand and sense strand of siRNA are modified nucleotides.

In some embodiments of the present disclosure, the modified nucleotide is selected from the group consisting of: 2'-methoxy nucleotide, 2'-fluoro nucleotide, 2'-deoxynucleotide, 2'-methoxyethyl nucleotide, 2'-amino nucleotide, 2'-alkyl nucleotide, 3'-methoxy nucleotide, but the present disclosure is not limited thereto.

In some embodiments of the present disclosure, the modified nucleotide is selected from a 2'-methoxy nucleotide or a 2'-fluoro nucleotide.

In some embodiments of the present disclosure, the modified nucleotide is a phosphate analog-modified nucleotide, well known to those skilled in the art, including, for example, *(E)*-vinylphosphate (*(E)*-VP)-modified nucleotide, *(Z)*-vinylphosphate (*(Z)*-VP)-modified nucleotide, phosphorothioate (Ps)-modified nucleotide, Sp-configured phosphorothioate-modified nucleotide, Rp-configured phosphorothioate-modified nucleotide (Anastasia Khvorova & Jonathan K. Watts, The chemical evolution of oligonucleotide therapies of clinical utility. Nature Biotechnology, 2017, 35(3): p. 238-248), or methanesulfonylaminophosphonate (MsPA)-modified nucleotide.

In some embodiments of the present disclosure, the nucleotide analog refers to a group that is capable of substituting nucleotides in a nucleic acid, but differs in base structure from adenine ribonucleotide, guanine ribonucleotide, cytosine ribonucleotide, uracil ribonucleotide or thymine deoxyribonucleotide, and such nucleotide analogs are known in the art and include, for example, locked nucleic acid (LNA), unlocked nucleic acid (UNA) or glycerol nucleic acid (GNA). In some embodiments of the present disclosure, the nucleotide analogue is a locked nucleic acid (LNA) or a glycerol nucleic acid (GNA).

In some embodiments of the present disclosure, all of the nucleotides of the sense strand and/or the antisense strand are modified nucleotides or nucleotide analogs.

In some embodiments of the present disclosure, each nucleotide in the sense strand and the antisense strand of the siRNA is independently a 2'-methoxy nucleotide, a 2'-fluoro nucleotide, or a 2'-deoxynucleotide.

In some embodiments of the present disclosure, any one of the following modifications of siRNA is provided:
1. A siRNA that inhibits target gene expression, the sense strand and antisense strand of the siRNA comprising one or more of a 2'-methoxy modified nucleotide, a 2'-fluoro modified nucleotide, a 5'-VP modified nucleotide, and comprising one or more phosphorothioate groups located between two adjacent nucleotides.
2. A siRNA for inhibiting the target gene expression, the siRNA comprises a sense strand and an antisense strand, wherein the sense strand is 17-21 continuous nucleotides in length, the antisense strand is 19-23 continuous nucleotides in length, the sense strand and the antisense strand comprise one or more of a 2'-methoxy modified nucleotide, a 2'-fluoro modified nucleotide, and a 5'-VP modified nucleotide, and comprise one or more phosphorothioate groups located between two adjacent nucleotides; at least one nucleotide at a position greater than 15 of the antisense strand in the 5' end to 3' end direction is mismatched to the target gene.
3. A siRNA for inhibiting the target gene expression, the siRNA comprises a sense strand and an antisense strand, wherein the sense strand is 17-21 continuous nucleotides in length, the antisense strand is 19-23 continuous nucleotides in length, the sense strand and the antisense strand comprise one or more of a 2'-methoxy modified nucleotide, a 2'-fluoro modified nucleotide, and a 5'-VP modified nucleotide, and comprise one or more phosphorothioate groups located between two adjacent nucleotides; at least one nucleotide at positions 19, 20, or 21 of the antisense strand in the 5' end to 3' end direction is mismatched to the target gene.
4. A siRNA for inhibiting the target gene expression, the siRNA comprises a sense strand and an antisense strand, wherein the sense strand is 17-21 continuous nucleotides in length, the antisense strand is 19-23 continuous nucleotides in length, the sense strand and the antisense strand comprise one or more of a 2'-methoxy modified nucleotide, a 2'-fluoro modified nucleotide, and a 5'-VP modified nucleotide, and comprise one or more phosphorothioate groups located between two adjacent nucleotides; the nucleotide at position 19 of the antisense strand in the 5' end to 3' end direction is mismatched to the target gene.
5. A siRNA for inhibiting the target gene expression, the siRNA comprises a sense strand and an antisense strand, wherein the sense strand is 17-21 continuous nucleotides in length, the antisense strand is 19-23 continuous nucleotides in length, the sense strand and the antisense strand comprise one or more of a 2'-methoxy modified nucleotide, a 2'-fluoro modified nucleotide, and a 5'-VP modified nucleotide, and comprise one or more phosphorothioate groups located between two adjacent nucleotides; the nucleotide at position 20 of the antisense strand in the 5' end to 3' end direction is mismatched to the target gene.
6. A siRNA for inhibiting the target gene expression, the siRNA comprises a sense strand and an antisense strand, wherein the sense strand is 17-21 continuous nucleotides in length, the antisense strand is 19-23 continuous nucleotides in length, the sense strand and the antisense strand comprise one or more of a 2'-methoxy modified nucleotide, a 2'-fluoro modified nucleotide, and a 5'-VP modified nucleotide, and comprise one or more phosphorothioate groups located between two adjacent nucleotides; the nucleotide at position 21 of the antisense strand in the 5' end to 3' end direction is mismatched to the target gene.
7. A siRNA for inhibiting the target gene expression, the siRNA comprises a sense strand and an antisense strand, wherein the sense strand is 17-21 continuous nucleotides in length, the antisense strand is 19-23 continuous nucleotides in length, the sense strand and the antisense strand comprise one or more of a 2'-methoxy modified nucleotide, a 2'-fluoro modified nucleotide, and a 5'-VP modified nucleotide, and comprise one or more phosphorothioate groups located between two adjacent nucleotides; and the nucleotides at positions 19 and 20 of the antisense strand in the 5' end to 3' end direction are mismatched to the target gene.
8. A siRNA for inhibiting the target gene expression, the siRNA comprises a sense strand and an antisense strand, wherein the sense strand is 17-21 continuous nucleotides in length, the antisense strand is 19-23 continuous nucleotides in length, the sense strand and the antisense strand comprise one or more of a 2'-methoxy modified nucleotide, a 2'-fluoro modified nucleotide, and a 5'-VP modified nucleotide, and comprise one or more phosphorothioate groups located between two adjacent nucleotides; and the nucleotides at positions 20 and 21 of the antisense strand in the 5' end to 3' end direction are mismatched to the target gene.
9. A siRNA for inhibiting the target gene expression, the siRNA comprises a sense strand and an antisense strand, wherein the sense strand is 17-21 continuous nucleotides in length, the antisense strand is 19-23 continuous nucleotides in length, the sense strand and the antisense strand comprise one or more of a 2'-methoxy modified nucleotide, a 2'-fluoro modified nucleotide, and a 5'-VP modified nucleotide, and comprise one or more phosphorothioate groups located between two adjacent nucleotides; and the nucleotides at positions 19 and 21 of the antisense strand in the 5' end to 3' end direction are mismatched to the target gene.
10. A siRNA for inhibiting the target gene expression, the siRNA comprises a sense strand and an antisense strand, wherein the sense strand is 17-21 continuous nucleotides in length, the antisense strand is 19-23 continuous nucleotides in length, the sense strand and the antisense strand comprise one or more of a 2'-methoxy modified nucleotide, a 2'-fluoro modified nucleotide, and a 5'-VP modified nucleotide, and comprise one or more phosphorothioate groups located between two adjacent nucleotides; and the nucleotides at positions 19, 20, and 21 of the antisense strand in the 5' end to 3' end direction are mismatched to the target gene.
11. A siRNA for inhibiting the target gene expression, the siRNA comprises a sense strand and an antisense strand, wherein the sense strand is 21 continuous nucleotides in length, the antisense strand is 23 continuous nucleotides in length, the sense strand and the antisense strand comprise one or more of a 2'-methoxy modified nucleotide, a 2'-fluoro modified nucleotide, and a 5'-VP modified nucleotide, and comprise one or more phosphorothioate groups located between two adjacent nucleotides; at least one nucleotide at positions 19, 20, or 21 of the antisense strand in the 5' end to 3' end direction is mismatched to the target gene.
12. A siRNA for inhibiting the target gene expression, the siRNA comprises a sense strand and an antisense strand, wherein the sense strand is 21 continuous nucleotides in length, the antisense strand is 23 continuous nucleotides in length, the sense strand and the antisense strand comprise one or more of a 2'-methoxy modified nucleotide, a 2'-fluoro modified nucleotide, and a 5'-VP modified nucleotide, and comprise one or more phosphorothioate groups located between two adjacent nucleotides; and the nucleotides at positions 19, 20, and 21 of the antisense strand in the 5' end to 3' end direction are mismatched to the target gene.
13. A siRNA for inhibiting the target gene expression, the siRNA comprises a sense strand and an antisense strand, wherein the sense strand is 21 continuous nucleotides in length, the antisense strand is 23 continuous nucleotides in length, the sense strand and the antisense strand comprise one or more of a 2'-methoxy modified nucleotide, a 2'-fluoro modified nucleotide, and a 5'-VP modified nucleotide, and comprise one or more phosphorothioate groups located between two adjacent nucleotides; at least one nucleotide at positions 19, 20, or 21 of the antisense strand in the 5' end to 3' end direction is mismatched to the target gene, the nucleotide of the antisense strand is identical to the nucleotide of the target gene at the mismatch position, and the nucleotide of the antisense strand and the nucleotide of the sense strand are complementary paired at the mismatch position.
14. A siRNA for inhibiting the target gene expression, the siRNA comprises a sense strand and an antisense strand, wherein the sense strand is 21 continuous nucleotides in length, the antisense strand is 23 continuous nucleotides in length, the sense strand and the antisense strand comprise one or more of a 2'-methoxy modified nucleotide, a 2'-fluoro modified nucleotide, and a 5'-VP modified nucleotide, and comprise one or more phosphorothioate groups located between two adjacent nucleotides; the nucleotides at positions 19, 20, and 21 of the antisense strand in the 5' end to 3' end direction are mismatched to the target gene, the nucleotides of the antisense strand are identical to the nucleotides of the target gene at the mismatch positions, and the nucleotides of the antisense strand and the nucleotides of the sense strand are complementary paired at the mismatch positions.
15. A siRNA for inhibiting the target gene expression, the siRNA comprises a sense strand and an antisense strand, the sense strand is 21 continuous nucleotides in length, the antisense strand is 23 continuous nucleotides in length, the sense strand and the antisense strand comprise one or more of a 2'-methoxy modified nucleotide, a 2'-fluoro modified nucleotide, and a 5'-VP modified nucleotide, and comprise one or more phosphorothioate groups located between two adjacent nucleotides; at least one nucleotide at positions 19, 20, or 21 of the antisense strand in the 5' end to 3' end direction is mismatched to the target gene, the nucleotide of the antisense strand is identical to the nucleotide of the target gene at the mismatch position, and the nucleotide of the antisense strand and the nucleotide of the sense strand are complementary paired at the mismatch position, an overhang of 2 nucleotides in length of the antisense strand comprises in the 5' to 3' direction: CU, GG, or GA.
16. A siRNA for inhibiting the target gene expression, the siRNA comprises a sense strand and an antisense strand, the sense strand is 21 continuous nucleotides in length, the antisense strand is 23 continuous nucleotides in length, the sense strand and the antisense strand comprise one or more of a 2'-methoxy modified nucleotide, a 2'-fluoro modified nucleotide, and a 5'-VP modified nucleotide, and comprise one or more phosphorothioate groups located between two adjacent nucleotides; at least one nucleotide at positions 19, 20, or 21 of the antisense strand in the 5' end to 3' end direction is mismatched to the target gene, the nucleotide of the antisense strand is identical to the nucleotide of the target gene at the mismatch position, and the nucleotide of the antisense strand and the nucleotide of the sense strand are complementary paired at the mismatch position, an overhang of 2 nucleotides in length of the antisense strand comprises in the 5' to 3' direction: CU, GG, or GA, the 5' end of the antisense strand in the siRNA forms a blunt end with the 3' end of the sense strand.
17. A siRNA for inhibiting the target gene expression, the siRNA comprises a sense strand and an antisense strand, wherein the sense strand is 21 continuous nucleotides in length, the antisense strand is 23 continuous nucleotides in length, the sense strand and the antisense strand comprise one or more of a 2'-methoxy modified nucleotide, a 2'-fluoro modified nucleotide, and a 5'-VP modified nucleotide, and comprise one or more phosphorothioate groups located between two adjacent nucleotides; at least one nucleotide at positions 19, 20, or 21 of the antisense strand in the 5' end to 3' end direction is mismatched to the target gene, the nucleotide of the antisense strand is identical to the nucleotide of the target gene at the mismatch position, and the nucleotide of the antisense strand and the nucleotide of the sense strand are complementary paired at the mismatch position, an overhang of 2 nucleotides in length of the antisense strand comprises in the 5' to 3' direction: CU, GG, or GA, the 5' end of the antisense strand in the siRNA forms a blunt end with the 3' end of the sense strand, and the antisense strand comprises a phosphorothioate group at one or more of the following internucleotide linkages: between positions 1 and 2, or between positions 2 and 3, wherein the positions are numbered from the 5' end.
18. A siRNA for inhibiting the target gene expression, the siRNA comprises a sense strand and an antisense strand, wherein the sense strand is 21 continuous nucleotides in length, the antisense strand is 23 continuous nucleotides in length, the sense strand and the antisense strand comprise one or more of a 2'-methoxy modified nucleotide, a 2'-fluoro modified nucleotide, and a 5'-VP modified nucleotide, and comprise one or more phosphorothioate groups located between two adjacent nucleotides; at least one nucleotide at positions 19, 20, or 21 of the antisense strand in the 5' end to 3' end direction is mismatched to the target gen, the nucleotide of the antisense strand is identical to the nucleotide of the target gene at the mismatch position, and the nucleotide of the antisense strand and the nucleotide of the sense strand are complementary paired at the mismatch position, an overhang of 2 nucleotides in length of the antisense strand comprises in the 5' to 3' direction: CU, GG, or GA, the 5' end of the antisense strand in the siRNA forms a blunt end with the 3' end of the sense strand, and the antisense strand comprises phosphorothioate groups at the following internucleotide linkages: between positions 1 and 2 numbered from the 5' end, between positions 2 and 3 numbered from the 5' end, between positions 1 and 2 numbered from the 3' end, and between positions 2 and 3 numbered from the 3' end.
19. A siRNA for inhibiting the target gene expression, the siRNA comprises a sense strand and an antisense strand, wherein the sense strand is 21 continuous nucleotides in length, the antisense strand is 23 continuous nucleotides in length, the sense strand and the antisense strand comprise one or more of a 2'-methoxy modified nucleotide, a 2'-fluoro modified nucleotide, and a 5'-VP modified nucleotide; at least one nucleotide at positions 19, 20, or 21 of the antisense strand in the 5' end to 3' end direction is mismatched to the target gene, the nucleotide of the antisense strand is identical to the nucleotide of the target gene at the mismatch position, and the nucleotide of the antisense strand and the nucleotide of the sense strand are complementary paired at the mismatch position, an overhang of 2 nucleotides in length of the antisense strand comprises in the 5' to 3' direction: CU, GG, or GA, the 5' end of the antisense strand in the siRNA forms a blunt end with the 3' end of the sense strand, and the antisense strand comprises phosphorothioate groups at the following internucleotide linkages: between positions 1 and 2 numbered from the 5' end, between positions 2 and 3 numbered from the 5' end, between positions 1 and 2 numbered from the 3' end, and between positions 2 and 3 numbered from the 3' end, and the nucleotide at position 1 of the antisense strand in the 5' end to 3' end direction is U or A.
20. A siRNA for inhibiting the target gene expression, the siRNA comprises a sense strand and an antisense strand, wherein the sense strand is 21 continuous nucleotides in length, the antisense strand is 23 continuous nucleotides in length, the sense strand and the antisense strand comprise one or more of a 2'-methoxy modified nucleotide, a 2'-fluoro modified nucleotide, and a 5'-VP modified nucleotide, and comprise one or more phosphorothioate groups located between two adjacent nucleotides; at least one nucleotide at positions 19, 20, or 21 of the antisense strand in the 5' end to 3' end direction is mismatched to the target gene, the nucleotide of the antisense strand is identical to the nucleotide of the target gene at the mismatch position, and the nucleotide of the antisense strand and the nucleotide of the sense strand are complementary paired at the mismatch position, an overhang of 2 nucleotides in length of the antisense strand comprises in the 5' to 3' direction: CU, GG, or GA, the 5' end of the antisense strand in the siRNA forms a blunt end with the 3' end of the sense strand, and the antisense strand comprises phosphorothioate groups at the following internucleotide linkages: between positions 1 and 2 numbered from the 5' end, between positions 2 and 3 numbered from the 5' end, between positions 1 and 2 numbered from the 3' end, and between positions 2 and 3 numbered from the 3' end, the nucleotide at position 1 of the antisense strand in the 5' end to 3' end direction is U or A, the nucleotide at position 2 of the antisense strand in the 5' end to 3' end direction is not U.
21. A siRNA for inhibiting the target gene expression, the siRNA comprises a sense strand and an antisense strand, wherein the sense strand is 21 continuous nucleotides in length, the antisense strand is 23 continuous nucleotides in length, the sense strand and the antisense strand comprise one or more of a 2'-methoxy modified nucleotide, a 2'-fluoro modified nucleotide, and a 5'-VP modified nucleotide, and comprise one or more phosphorothioate groups located between two adjacent nucleotides; at least one nucleotide at positions 19, 20, or 21 of the antisense strand in the 5' end to 3' end direction is mismatched to the target gene, the nucleotide of the antisense strand is identical to the nucleotide of the target gene at the mismatch position, and the nucleotide of the antisense strand and the nucleotide of the sense strand are complementary paired at the mismatch position, an overhang of 2 nucleotides in length of the antisense strand comprises in the 5' to 3' direction: CU, GG, or GA, the 5' end of the antisense strand in the siRNA forms a blunt end with the 3' end of the sense strand, and the antisense strand comprises phosphorothioate groups at the following internucleotide linkages: between positions 1 and 2 numbered from the 5' end, between positions 2 and 3 numbered from the 5' end, between positions 1 and 2 numbered from the 3' end, and between positions 2 and 3 numbered from the 3' end, and the nucleotide at position 1 of the antisense strand in the 5' end to 3' end direction is U or A, the nucleotide at position 2 of the antisense strand in the 5' end to 3' end direction is not U, the antisense strand comprises: (a) no more than six nucleotides modified with a 2'-fluoro group at the 2'-position of the ribose sugar moiety, and (b) the remainder of the nucleotides modified with a 2'-methoxy group at the 2'- position of the ribose sugar moiety.
22. A siRNA for inhibiting the target gene expression, the siRNA comprises a sense strand and an antisense strand, wherein the sense strand is 21 continuous nucleotides in length, the antisense strand is 23 continuous nucleotides in length, the sense strand and the antisense strand comprise one or more of a 2'-methoxy modified nucleotide, a 2'-fluoro modified nucleotide, and a 5'-VP modified nucleotide, and comprise one or more phosphorothioate groups located between two adjacent nucleotides; at least one nucleotide at positions 19, 20, or 21 of the antisense strand in the 5' end to 3' end direction is mismatched to the target gene, the nucleotide of the antisense strand is identical to the nucleotide of the target gene at the mismatch position, and the nucleotide of the antisense strand and the nucleotide of the sense strand are complementary paired at the mismatch position, an overhang of 2 nucleotides in length of the antisense strand comprises in the 5' to 3' direction: CU, GG, or GA, the 5' end of the antisense strand in the siRNA forms a blunt end with the 3' end of the sense strand, and the antisense strand comprises phosphorothioate groups at the following internucleotide linkages: between positions 1 and 2 numbered from the 5' end, between positions 2 and 3 numbered from the 5' end, between positions 1 and 2 numbered from the 3' end, and between positions 2 and 3 numbered from the 3' end, and the nucleotide at position 1 of the antisense strand in the 5' end to 3' end direction is U or A, the nucleotide at position 2 of the antisense strand in the 5' end to 3' end direction is not U, the antisense strand comprises: (a) five to six nucleotides modified with a 2'-fluoro group at the 2'-position of the ribose sugar moiety, and (b) the remainder of the nucleotides of the antisense strand modified with a 2'-methoxy group at the 2'- position of the ribose sugar moiety.
23. A siRNA for inhibiting the target gene expression, the siRNA comprises a sense strand and an antisense strand, wherein the sense strand is 21 continuous nucleotides in length, the antisense strand is 23 continuous nucleotides in length, the sense strand and the antisense strand comprise one or more of a 2'-methoxy modified nucleotide, a 2'-fluoro modified nucleotide, and a 5'-VP modified nucleotide, and comprise one or more phosphorothioate groups located between two adjacent nucleotides; at least one nucleotide at positions 19, 20, or 21 of the antisense strand in the 5' end to 3' end direction is mismatched to the target gene, the nucleotide of the antisense strand is identical to the nucleotide of the target gene at the mismatch position, and the nucleotide of the antisense strand and the nucleotide of the sense strand are complementary paired at the mismatch position, an overhang of 2 nucleotides in length of the antisense strand comprises in the 5' to 3' direction: CU, GG, or GA, the 5' end of the antisense strand in the siRNA forms a blunt end with the 3' end of the sense strand, and the antisense strand comprises phosphorothioate groups at the following internucleotide linkages: between positions 1 and 2 numbered from the 5' end, between positions 2 and 3 numbered from the 5' end, between positions 1 and 2 numbered from the 3' end, and between positions 2 and 3 numbered from the 3' end, and the nucleotide at position 1 of the antisense strand in the 5' end to 3' end direction is U or A, the nucleotide at position 2 of the antisense strand in the 5' end to 3' end direction is not U, the nucleotides at positions 2, 4, 6, 14, and 16 of the antisense strand in the 5' end to 3' end direction are 2'-fluoro modified nucleotides, and the remainder of the nucleotides of the antisense strand modified with a 2'-methoxy group at the 2'- position of the ribose sugar moiety.
24. A siRNA for inhibiting the target gene expression, the siRNA comprises a sense strand and an antisense strand, wherein the sense strand is 21 continuous nucleotides in length, the antisense strand is 23 continuous nucleotides in length, the sense strand and the antisense strand comprise one or more of a 2'-methoxy modified nucleotide, a 2'-fluoro modified nucleotide, and a 5'-VP modified nucleotide, and comprise one or more phosphorothioate groups located between two adjacent nucleotides; at least one nucleotide at positions 19, 20, or 21 of the antisense strand in the 5' end to 3' end direction is mismatched to the target gene, the nucleotide of the antisense strand is identical to the nucleotide of the target gene at the mismatch position, and the nucleotide of the antisense strand and the nucleotide of the sense strand are complementary paired at the mismatch position, an overhang of 2 nucleotides in length of the antisense strand comprises in the 5' to 3' direction: CU, GG, or GA, the 5' end of the antisense strand in the siRNA forms a blunt end with the 3' end of the sense strand, and the antisense strand comprises phosphorothioate groups at the following internucleotide linkages: between positions 1 and 2 numbered from the 5' end, between positions 2 and 3 numbered from the 5' end, between positions 1 and 2 numbered from the 3' end, and between positions 2 and 3 numbered from the 3' end, and the nucleotide at position 1 of the antisense strand in the 5' end to 3' end direction is U or A, the nucleotide at position 2 of the antisense strand in the 5' end to 3' end direction is not U, the nucleotides at positions 2, 4, 6, 14, and 16 of the antisense strand in the 5' end to 3' end direction are 2'-fluoro modified nucleotides, and the remainder of the nucleotides of the antisense strand modified with a 2'-methoxy group at the 2'- position of the ribose sugar moiety, and the sense strand comprises no more than six 2'-fluoro modified nucleotides, and the remainder of the nucleotides of the sense strand are all 2'-methoxy modified nucleotides.
25. A siRNA for inhibiting the target gene expression, the siRNA comprises a sense strand and an antisense strand, wherein the sense strand is 21 continuous nucleotides in length, the antisense strand is 23 continuous nucleotides in length, the sense strand and the antisense strand comprise one or more of a 2'-methoxy modified nucleotide, a 2'-fluoro modified nucleotide, and a 5'-VP modified nucleotide, and comprise one or more phosphorothioate groups located between two adjacent nucleotides; at least one nucleotide at positions 19, 20, or 21 of the antisense strand in the 5' end to 3' end direction is mismatched to the target gene, the nucleotide of the antisense strand is identical to the nucleotide of the target gene at the mismatch position, and the nucleotide of the antisense strand and the nucleotide of the sense strand are complementary paired at the mismatch position, an overhang of 2 nucleotides in length of the antisense strand comprises in the 5' to 3' direction: CU, GG, or GA, the 5' end of the antisense strand in the siRNA forms a blunt end with the 3' end of the sense strand, and the antisense strand comprises phosphorothioate groups at the following internucleotide linkages: between positions 1 and 2 numbered from the 5' end, between positions 2 and 3 numbered from the 5' end, between positions 1 and 2 numbered from the 3' end, and between positions 2 and 3 numbered from the 3' end, and the nucleotide at position 1 of the antisense strand in the 5' end to 3' end direction is U or A, the nucleotide at position 2 of the antisense strand in the 5' end to 3' end direction is not U, the nucleotides at positions 2, 4, 6, 14, and 16 of the antisense strand in the 5' end to 3' end direction are 2'-fluoro modified nucleotides, and the remainder of the nucleotides of the antisense strand modified with a 2'-methoxy group at the 2'- position of the ribose sugar moiety, and the sense strand comprises five to six 2'-fluoro modified nucleotides, and the remainder of the nucleotides of the sense strand are all 2'-methoxy modified nucleotides.
26. A siRNA for inhibiting the target gene expression, the siRNA comprises a sense strand and an antisense strand, wherein the sense strand is 21 continuous nucleotides in length, the antisense strand is 23 continuous nucleotides in length, the sense strand and the antisense strand comprise one or more of a 2'-methoxy modified nucleotide, a 2'-fluoro modified nucleotide, and a 5'-VP modified nucleotide, and comprise one or more phosphorothioate groups located between two adjacent nucleotides; at least one nucleotide at positions 19, 20, or 21 of the antisense strand in the 5' end to 3' end direction is mismatched to the target gene, the nucleotide of the antisense strand is identical to the nucleotide of the target gene at the mismatch position, and the nucleotide of the antisense strand and the nucleotide of the sense strand are complementary paired at the mismatch position, an overhang of 2 nucleotides in length of the antisense strand comprises in the 5' to 3' direction: CU, GG, or GA, the 5' end of the antisense strand in the siRNA forms a blunt end with the 3' end of the sense strand, and the antisense strand comprises phosphorothioate groups at the following internucleotide linkages: between positions 1 and 2 numbered from the 5' end, between positions 2 and 3 numbered from the 5' end, between positions 1 and 2 numbered from the 3' end, and between positions 2 and 3 numbered from the 3' end, and the nucleotide at position 1 of the antisense strand in the 5' end to 3' end direction is U or A, the nucleotide at position 2 of the antisense strand in the 5' end to 3' end direction is not U, the nucleotides at positions 2, 4, 6, 14, and 16 of the antisense strand in the 5' end to 3' end direction are 2'-fluoro modified nucleotides, and the remainder of the nucleotides of the antisense strand modified with a 2'-methoxy group at the 2'- position of the ribose sugar moiety, the nucleotides at positions 7, 9, 10, 11, and 13 of the 5' to 3' direction of the sense strand are 2'-fluoro modified nucleotides, and the remainder of the nucleotides of the sense strand are all 2'-methoxy modified nucleotides.
27. A siRNA for inhibiting the target gene expression, the siRNA comprises a sense strand and an antisense strand, wherein the sense strand is 21 continuous nucleotides in length, the antisense strand is 23 continuous nucleotides in length, the sense strand and the antisense strand comprise one or more of a 2'-methoxy modified nucleotide, a 2'-fluoro modified nucleotide, and a 5'-VP modified nucleotide, and comprise one or more phosphorothioate groups located between two adjacent nucleotides; at least one nucleotide at positions 19, 20, or 21 of the antisense strand in the 5' end to 3' end direction is mismatched to the target gene, the nucleotide of the antisense strand is identical to the nucleotide of the target gene at the mismatch position, and the nucleotide of the antisense strand and the nucleotide of the sense strand are complementary paired at the mismatch position, an overhang of 2 nucleotides in length of the antisense strand comprises in the 5' to 3' direction: CU, GG, or GA, the 5' end of the antisense strand in the siRNA forms a blunt end with the 3' end of the sense strand, and the antisense strand comprises phosphorothioate groups at the following internucleotide linkages: between positions 1 and 2 numbered from the 5' end, between positions 2 and 3 numbered from the 5' end, between positions 1 and 2 numbered from the 3' end, and between positions 2 and 3 numbered from the 3' end, and the nucleotide at position 1 of the antisense strand in the 5' end to 3' end direction is U or A, the nucleotide at position 2 of the antisense strand in the 5' end to 3' end direction is not U, the nucleotides at positions 2, 6, 10, 14, and 16 of the antisense strand in the 5' end to 3' end direction are 2'-fluoro modified nucleotides, and the remainder of the nucleotides of the antisense strand modified with a 2'-methoxy group at the 2'- position of the ribose sugar moiety.
28. A siRNA for inhibiting the target gene expression, the siRNA comprises a sense strand and an antisense strand, wherein the sense strand is 21 continuous nucleotides in length, the antisense strand is 23 continuous nucleotides in length, the sense strand and the antisense strand comprise one or more of a 2'-methoxy modified nucleotide, a 2'-fluoro modified nucleotide, and a 5'-VP modified nucleotide, and comprise one or more phosphorothioate groups located between two adjacent nucleotides; at least one nucleotide at positions 19, 20, or 21 of the antisense strand in the 5' end to 3' end direction is mismatched to the target gene, the nucleotide of the antisense strand is identical to the nucleotide of the target gene at the mismatch position, and the nucleotide of the antisense strand and the nucleotide of the sense strand are complementary paired at the mismatch position, an overhang of 2 nucleotides in length of the antisense strand comprises in the 5' to 3' direction: CU, GG, or GA, the 5' end of the antisense strand in the siRNA forms a blunt end with the 3' end of the sense strand, and the antisense strand comprises phosphorothioate groups at the following internucleotide linkages: between positions 1 and 2 numbered from the 5' end, between positions 2 and 3 numbered from the 5' end, between positions 1 and 2 numbered from the 3' end, and between positions 2 and 3 numbered from the 3' end, and the nucleotide at position 1 of the antisense strand in the 5' end to 3' end direction is U or A, the nucleotide at position 2 of the antisense strand in the 5' end to 3' end direction is not U, the nucleotides at positions 2, 6, 10, 14, and 16 of the antisense strand in the 5' end to 3' end direction are 2'-fluoro modified nucleotides, and the remainder of the nucleotides of the antisense strand modified with a 2'-methoxy group at the 2'- position of the ribose sugar moiety, and the sense strand comprises no more than six 2'-fluoro modified nucleotides, and the remainder of the nucleotides of the sense strand are all 2'-methoxy modified nucleotides.
29. A siRNA for inhibiting the target gene expression, the siRNA comprises a sense strand and an antisense strand, wherein the sense strand is 21 continuous nucleotides in length, the antisense strand is 23 continuous nucleotides in length, the sense strand and the antisense strand comprise one or more of a 2'-methoxy modified nucleotide, a 2'-fluoro modified nucleotide, and a 5'-VP modified nucleotide, and comprise one or more phosphorothioate groups located between two adjacent nucleotides; at least one nucleotide at positions 19, 20, or 21 of the antisense strand in the 5' end to 3' end direction is mismatched to the target gene, the nucleotide of the antisense strand is identical to the nucleotide of the target gene at the mismatch position, and the nucleotide of the antisense strand and the nucleotide of the sense strand are complementary paired at the mismatch position, an overhang of 2 nucleotides in length of the antisense strand comprises in the 5' to 3' direction: CU, GG, or GA, the 5' end of the antisense strand in the siRNA forms a blunt end with the 3' end of the sense strand, and the antisense strand comprises phosphorothioate groups at the following internucleotide linkages: between positions 1 and 2 numbered from the 5' end, between positions 2 and 3 numbered from the 5' end, between positions 1 and 2 numbered from the 3' end, and between positions 2 and 3 numbered from the 3' end, and the nucleotide at position 1 of the antisense strand in the 5' end to 3' end direction is U or A, the nucleotide at position 2 of the antisense strand in the 5' end to 3' end direction is not U, the nucleotides at positions 2, 6, 10, 14, and 16 of the antisense strand in the 5' end to 3' end direction are 2'-fluoro modified nucleotides, and the remainder of the nucleotides of the antisense strand modified with a 2'-methoxy group at the 2'- position of the ribose sugar moiety, and the sense strand comprises five to six 2'-fluoro modified nucleotides, and the remainder of the nucleotides of the sense strand are all 2'-methoxy modified nucleotides.
30. A siRNA for inhibiting the target gene expression, the siRNA comprises a sense strand and an antisense strand, wherein the sense strand is 21 continuous nucleotides in length, the antisense strand is 23 continuous nucleotides in length, the sense strand and the antisense strand comprise one or more of a 2'-methoxy modified nucleotide, a 2'-fluoro modified nucleotide, and a 5'-VP modified nucleotide, and comprise one or more phosphorothioate groups located between two adjacent nucleotides; at least one nucleotide at positions 19, 20, or 21 of the antisense strand in the 5' end to 3' end direction is mismatched to the target gene, the nucleotide of the antisense strand is identical to the nucleotide of the target gene at the mismatch position, and the nucleotide of the antisense strand and the nucleotide of the sense strand are complementary paired at the mismatch position, an overhang of 2 nucleotides in length of the antisense strand comprises in the 5' to 3' direction: CU, GG, or GA, the 5' end of the antisense strand in the siRNA forms a blunt end with the 3' end of the sense strand, and the antisense strand comprises phosphorothioate groups at the following internucleotide linkages: between positions 1 and 2 numbered from the 5' end, between positions 2 and 3 numbered from the 5' end, between positions 1 and 2 numbered from the 3' end, and between positions 2 and 3 numbered from the 3' end, and the nucleotide at position 1 of the antisense strand in the 5' end to 3' end direction is U or A, the nucleotide at position 2 of the antisense strand in the 5' end to 3' end direction is not U, the nucleotides at positions 2, 6, 10, 14, and 16 of the antisense strand in the 5' end to 3' end direction are 2'-fluoro modified nucleotides, and the remainder of the nucleotides of the antisense strand modified with a 2'-methoxy group at the 2'- position of the ribose sugar moiety, the nucleotides at positions 3, 7, 9, 10, and 11 of the 5' to 3' direction of the sense strand are 2'-fluoro modified nucleotides, and the remainder of the nucleotides of the sense strand are all 2'-methoxy modified nucleotides.
31. A siRNA for inhibiting the target gene expression, the siRNA comprises a sense strand and an antisense strand, wherein the sense strand is 21 continuous nucleotides in length, the antisense strand is 23 continuous nucleotides in length, the sense strand and the antisense strand comprise one or more of a 2'-methoxy modified nucleotide, a 2'-fluoro modified nucleotide, and a 5'-VP modified nucleotide, and comprise one or more phosphorothioate groups located between two adjacent nucleotides; at least one nucleotide at positions 19, 20, or 21 of the antisense strand in the 5' end to 3' end direction is mismatched to the target gene, the nucleotide of the antisense strand is identical to the nucleotide of the target gene at the mismatch position, and the nucleotide of the antisense strand and the nucleotide of the sense strand are complementary paired at the mismatch position, an overhang of 2 nucleotides in length of the antisense strand comprises in the 5' to 3' direction: CU, GG, or GA, the 5' end of the antisense strand in the siRNA forms a blunt end with the 3' end of the sense strand, and the antisense strand comprises phosphorothioate groups at the following internucleotide linkages: between positions 1 and 2 numbered from the 5' end, between positions 2 and 3 numbered from the 5' end, between positions 1 and 2 numbered from the 3' end, and between positions 2 and 3 numbered from the 3' end, and the nucleotide at position 1 of the antisense strand in the 5' end to 3' end direction is U or A, the nucleotide at position 2 of the antisense strand in the 5' end to 3' end direction is not U, the nucleotides at positions 2, 6, 14, 16, and 22 of the antisense strand in the 5' end to 3' end direction are 2'-fluoro modified nucleotides, and the remainder of the nucleotides of the antisense strand modified with a 2'-methoxy group at the 2'- position of the ribose sugar moiety.
32. A siRNA for inhibiting the target gene expression, the siRNA comprises a sense strand and an antisense strand, wherein the sense strand is 21 continuous nucleotides in length, the antisense strand is 23 continuous nucleotides in length, the sense strand and the antisense strand comprise one or more of a 2'-methoxy modified nucleotide, a 2'-fluoro modified nucleotide, and a 5'-VP modified nucleotide, and comprise one or more phosphorothioate groups located between two adjacent nucleotides; at least one nucleotide at positions 19, 20, or 21 of the antisense strand in the 5' end to 3' end direction is mismatched to the target gene, the nucleotide of the antisense strand is identical to the nucleotide of the target gene at the mismatch position, and the nucleotide of the antisense strand and the nucleotide of the sense strand are complementary paired at the mismatch position, an overhang of 2 nucleotides in length of the antisense strand comprises in the 5' to 3' direction: CU, GG, or GA, the 5' end of the antisense strand in the siRNA forms a blunt end with the 3' end of the sense strand, and the antisense strand comprises phosphorothioate groups at the following internucleotide linkages: between positions 1 and 2 numbered from the 5' end, between positions 2 and 3 numbered from the 5' end, between positions 1 and 2 numbered from the 3' end, and between positions 2 and 3 numbered from the 3' end, and the nucleotide at position 1 of the antisense strand in the 5' end to 3' end direction is U or A, the nucleotide at position 2 of the antisense strand in the 5' end to 3' end direction is not U, the nucleotides at positions 2, 6, 14, 16, and 22 of the antisense strand in the 5' end to 3' end direction are 2'-fluoro modified nucleotides, and the remainder of the nucleotides of the antisense strand modified with a 2'-methoxy group at the 2'- position of the ribose sugar moiety, and the sense strand comprises no more than six 2'-fluoro modified nucleotides, and the remainder of the nucleotides of the sense strand are all 2'-methoxy modified nucleotides.
33. A siRNA for inhibiting the target gene expression, the siRNA comprises a sense strand and an antisense strand, wherein the sense strand is 21 continuous nucleotides in length, the antisense strand is 23 continuous nucleotides in length, the sense strand and the antisense strand comprise one or more of a 2'-methoxy modified nucleotide, a 2'-fluoro modified nucleotide, and a 5'-VP modified nucleotide, and comprise one or more phosphorothioate groups located between two adjacent nucleotides; at least one nucleotide at positions 19, 20, or 21 of the antisense strand in the 5' end to 3' end direction is mismatched to the target gene, the nucleotide of the antisense strand is identical to the nucleotide of the target gene at the mismatch position, and the nucleotide of the antisense strand and the nucleotide of the sense strand are complementary paired at the mismatch position, an overhang of 2 nucleotides in length of the antisense strand comprises in the 5' to 3' direction: CU, GG, or GA, the 5' end of the antisense strand in the siRNA forms a blunt end with the 3' end of the sense strand, and the antisense strand comprises phosphorothioate groups at the following internucleotide linkages: between positions 1 and 2 numbered from the 5' end, between positions 2 and 3 numbered from the 5' end, between positions 1 and 2 numbered from the 3' end, and between positions 2 and 3 numbered from the 3' end, and the nucleotide at position 1 of the antisense strand in the 5' end to 3' end direction is U or A, the nucleotide at position 2 of the antisense strand in the 5' end to 3' end direction is not U, the nucleotides at positions 2, 6, 14, 16, and 22 of the antisense strand in the 5' end to 3' end direction are 2'-fluoro modified nucleotides, and the remainder of the nucleotides of the antisense strand modified with a 2'-methoxy group at the 2'- position of the ribose sugar moiety, and the sense strand comprises five to six 2'-fluoro modified nucleotides, and the remainder of the nucleotides of the sense strand are all 2'-methoxy modified nucleotides.
34. A siRNA for inhibiting the target gene expression, the siRNA comprises a sense strand and an antisense strand, wherein the sense strand is 21 continuous nucleotides in length, the antisense strand is 23 continuous nucleotides in length, the sense strand and the antisense strand comprise one or more of a 2'-methoxy modified nucleotide, a 2'-fluoro modified nucleotide, and a 5'-VP modified nucleotide, and comprise one or more phosphorothioate groups located between two adjacent nucleotides; at least one nucleotide at positions 19, 20, or 21 of the antisense strand in the 5' end to 3' end direction is mismatched to the target gene, the nucleotide of the antisense strand is identical to the nucleotide of the target gene at the mismatch position, and the nucleotide of the antisense strand and the nucleotide of the sense strand are complementary paired at the mismatch position, an overhang of 2 nucleotides in length of the antisense strand comprises in the 5' to 3' direction: CU, GG, or GA, the 5' end of the antisense strand in the siRNA forms a blunt end with the 3' end of the sense strand, and the antisense strand comprises phosphorothioate groups at the following internucleotide linkages: between positions 1 and 2 numbered from the 5' end, between positions 2 and 3 numbered from the 5' end, between positions 1 and 2 numbered from the 3' end, and between positions 2 and 3 numbered from the 3' end, and the nucleotide at position 1 of the antisense strand in the 5' end to 3' end direction is U or A, the nucleotide at position 2 of the antisense strand in the 5' end to 3' end direction is not U, the nucleotides at positions 2, 6, 14, 16, and 22 of the antisense strand in the 5' end to 3' end direction are 2'-fluoro modified nucleotides, and the remainder of the nucleotides of the antisense strand modified with a 2'-methoxy group at the 2'- position of the ribose sugar moiety, the nucleotides at positions 7, 9, 10, 11, and 13 of the 5' to 3' direction of the sense strand are 2'-fluoro modified nucleotides, and the remainder of the nucleotides of the asense strand are all 2'-methoxy modified nucleotides.
35. A siRNA for inhibiting the target gene expression, the siRNA comprises a sense strand and an antisense strand, wherein the sense strand is 21 continuous nucleotides in length, the antisense strand is 23 continuous nucleotides in length, the sense strand and the antisense strand comprise one or more of a 2'-methoxy modified nucleotide, a 2'-fluoro modified nucleotide, and a 5'-VP modified nucleotide, and comprise one or more phosphorothioate groups located between two adjacent nucleotides; at least one nucleotide at positions 19, 20, or 21 of the antisense strand in the 5' end to 3' end direction is mismatched to the target gene, the nucleotide of the antisense strand is identical to the nucleotide of the target gene at the mismatch position, and the nucleotide of the antisense strand and the nucleotide of the sense strand are complementary paired at the mismatch position, an overhang of 2 nucleotides in length of the antisense strand comprises in the 5' to 3' direction: CU, GG, or GA, the 5' end of the antisense strand in the siRNA forms a blunt end with the 3' end of the sense strand, and the antisense strand comprises phosphorothioate groups at the following internucleotide linkages: between positions 1 and 2 numbered from the 5' end, between positions 2 and 3 numbered from the 5' end, between positions 1 and 2 numbered from the 3' end, and between positions 2 and 3 numbered from the 3' end, and the nucleotide at position 1 of the antisense strand in the 5' end to 3' end direction is U or A, the nucleotide at position 2 of the antisense strand in the 5' end to 3' end direction is not U, the nucleotides at positions 2, 6, 14, 16 and 22 of the antisense strand in the 5' end to 3' end direction are 2'-fluoro modified nucleotides, and the remainder of the nucleotides of the antisense strand modified with a 2'-methoxy group at the 2'- position of the ribose sugar moiety, the nucleotides at positions 7, 9, 10, 11, 13 and 17 of the 5' to 3' direction of the sense strand are 2'-fluoro modified nucleotides, and the remainder of the nucleotides of the sense strand are all 2'-methoxy modified nucleotides.
36. A siRNA for inhibiting the target gene expression, the siRNA comprises a sense strand and an antisense strand, wherein the sense strand is 21 continuous nucleotides in length, the antisense strand is 23 continuous nucleotides in length, the sense strand and the antisense strand comprise one or more of a 2'-methoxy modified nucleotide, a 2'-fluoro modified nucleotide, and a 5'-VP modified nucleotide, and comprise one or more phosphorothioate groups located between two adjacent nucleotides; at least one nucleotide at positions 19, 20, or 21 of the antisense strand in the 5' end to 3' end direction is mismatched to the target gene, the nucleotide of the antisense strand is identical to the nucleotide of the target gene at the mismatch position, and the nucleotide of the antisense strand and the nucleotide of the sense strand are complementary paired at the mismatch position, an overhang of 2 nucleotides in length of the antisense strand comprises in the 5' to 3' direction: CU, GG, or GA, the 5' end of the antisense strand in the siRNA forms a blunt end with the 3' end of the sense strand, and the antisense strand comprises phosphorothioate groups at the following internucleotide linkages: between positions 1 and 2 numbered from the 5' end, between positions 2 and 3 numbered from the 5' end, between positions 1 and 2 numbered from the 3' end, and between positions 2 and 3 numbered from the 3' end, and the nucleotide at position 1 of the antisense strand in the 5' end to 3' end direction is U or A, the nucleotide at position 2 of the antisense strand in the 5' end to 3' end direction is not U, the nucleotides at positions 2, 6, 14, 16 and 22 of the antisense strand in the 5' end to 3' end direction are 2'-fluoro modified nucleotides, the remainder of the nucleotides of the antisense strand modified with a 2'-methoxy group at the 2'- position of the ribose sugar moiety, and the nucleotide at position 1 of the antisense strand in the 5' end to 3' end direction further has a 5'-VP modification, preferably a 5'-(E)-VP modification.
37. A siRNA for inhibiting the target gene expression, the siRNA comprises a sense strand and an antisense strand, wherein the sense strand is 21 continuous nucleotides in length, the antisense strand is 23 continuous nucleotides in length, the sense strand and the antisense strand include one or more of a 2'-methoxy modified nucleotide, a 2'-fluoro modified nucleotide, and a 5'-VP modified nucleotide, and comprise one or more phosphorothioate groups located between two adjacent nucleotides; at least one nucleotide at positions 19, 20, or 21 of the antisense strand in the 5' end to 3' end direction is mismatched to the target gene, the nucleotide of the antisense strand is identical to the nucleotide of the target gene at the mismatch position, and the nucleotide of the antisense strand and the nucleotide of the sense strand are complementary paired at the mismatch position, an overhang of 2 nucleotides in length of the antisense strand comprises in the 5' to 3' direction: CU, GG, or GA, the 5' end of the antisense strand in the siRNA forms a blunt end with the 3' end of the sense strand, and the antisense strand comprises phosphorothioate groups at the following internucleotide linkages: between positions 1 and 2 numbered from the 5' end, between positions 2 and 3 numbered from the 5' end, between positions 1 and 2 numbered from the 3' end, and between positions 2 and 3 numbered from the 3' end, and the nucleotide at position 1 of the antisense strand in the 5' end to 3' end direction is U or A, the nucleotide at position 2 of the antisense strand in the 5' end to 3' end direction is not U, the nucleotides at positions 2, 6, 14, 16, and 22 of the antisense strand in the 5' end to 3' end direction are 2'-fluoro modified nucleotides, the remainder of the nucleotides of the antisense strand modified with a 2'-methoxy group at the 2'- position of the ribose sugar moiety, and the nucleotide at position 1 of the antisense strand in the 5' end to 3' end direction further has a 5'-VP modification, preferably a 5'-(E)-VP modification, the sense strand comprises no more than six 2'-fluoro modified nucleotides; and the remainder of the nucleotides of the sense strand are all 2'-methoxy modified nucleotides.
38. A siRNA for inhibiting the target gene expression, the siRNA comprises a sense strand and an antisense strand, wherein the sense strand is 21 continuous nucleotides in length, the antisense strand is 23 continuous nucleotides in length, the sense strand and the antisense strand comprise one or more of a 2'-methoxy modified nucleotide, a 2'-fluoro modified nucleotide, and a 5'-VP modified nucleotide, and comprise one or more phosphorothioate groups located between two adjacent nucleotides; at least one nucleotide at positions 19, 20, or 21 of the antisense strand in the 5' end to 3' end direction is mismatched to the target gene, the nucleotide of the antisense strand is identical to the nucleotide of the target gene at the mismatch position, and the nucleotide of the antisense strand and the nucleotide of the sense strand are complementary paired at the mismatch position, an overhang of 2 nucleotides in length of the antisense strand comprises in the 5' to 3' direction: CU, GG, or GA, the 5' end of the antisense strand in the siRNA forms a blunt end with the 3' end of the sense strand, and the antisense strand comprises phosphorothioate groups at the following internucleotide linkages: between positions 1 and 2 numbered from the 5' end, between positions 2 and 3 numbered from the 5' end, between positions 1 and 2 numbered from the 3' end, and between positions 2 and 3 numbered from the 3' end, and the nucleotide at position 1 of the antisense strand in the 5' end to 3' end direction is U or A, the nucleotide at position 2 of the antisense strand in the 5' end to 3' end direction is not U, the nucleotides at positions 2, 6, 14, 16, and 22 of the antisense strand in the 5' end to 3' end direction are 2'-fluoro modified nucleotides, the remainder of the nucleotides of the antisense strand modified with a 2'-methoxy group at the 2'- position of the ribose sugar moiety, and the nucleotide at position 1 of the antisense strand in the 5' end to 3' end direction further has a 5'-VP modification, preferably a 5'-(E)-VP modification, the sense strand comprises five to six 2'-fluoro modified nucleotides; and the remainder of the nucleotides of the sense strand are all 2'-methoxy modified nucleotides.
39. A siRNA for inhibiting the target gene expression, the siRNA comprises a sense strand and an antisense strand, wherein the sense strand is 21 continuous nucleotides in length, the antisense strand is 23 continuous nucleotides in length, the sense strand and the antisense strand comprise one or more of a 2'-methoxy modified nucleotide, a 2'-fluoro modified nucleotide, and a 5'-VP modified nucleotide, and comprise one or more phosphorothioate groups located between two adjacent nucleotides; at least one nucleotide at positions 19, 20, or 21 of the antisense strand in the 5' end to 3' end direction is mismatched to the target gene, the nucleotide of the antisense strand is identical to the nucleotide of the target gene at the mismatch position, and the nucleotide of the antisense strand and the nucleotide of the sense strand are complementary paired at the mismatch position, an overhang of 2 nucleotides in length of the antisense strand comprises in the 5' to 3' direction: CU, GG, or GA, the 5' end of the antisense strand in the siRNA forms a blunt end with the 3' end of the sense strand, and the antisense strand comprises phosphorothioate groups at the following internucleotide linkages: between positions 1 and 2 numbered from the 5' end, between positions 2 and 3 numbered from the 5' end, between positions 1 and 2 numbered from the 3' end, and between positions 2 and 3 numbered from the 3' end, and the nucleotide at position 1 of the antisense strand in the 5' end to 3' end direction is U or A, the nucleotide at position 2 of the antisense strand in the 5' end to 3' end direction is not U, the nucleotides at positions 2, 6, 14, 16, and 22 of the antisense strand in the 5' end to 3' end direction are 2'-fluoro modified nucleotides, the remainder of the nucleotides of the antisense strand modified with a 2'-methoxy group at the 2'- position of the ribose sugar moiety, and the nucleotide at position 1 of the antisense strand in the 5' end to 3' end direction further has a 5'-VP modification, preferably a 5'-(E)-VP modification, the nucleotides at positions 7, 9, 10, 11, and 13 of the sense strand in the 5' to 3' direction are 2'-fluoro modified nucleotides, and the remainder of the nucleotides of the sense strand are 2'-methoxy modified nucleotides.
40. A siRNA for inhibiting the target gene expression, the siRNA comprises a sense strand and an antisense strand, wherein the sense strand is 21 continuous nucleotides in length, the antisense strand is 23 continuous nucleotides in length, the sense strand and the antisense strand comprise one or more of a 2'-methoxy modified nucleotide, a 2'-fluoro modified nucleotide, and a 5'-VP modified nucleotide, and comprise one or more phosphorothioate groups located between two adjacent nucleotides; at least one nucleotide at positions 19, 20, or 21 of the antisense strand in the 5' end to 3' end direction is mismatched to the target gene, the nucleotide of the antisense strand is identical to the nucleotide of the target gene at the mismatch position, and the nucleotide of the antisense strand and the nucleotide of the sense strand are complementary paired at the mismatch position, an overhang of 2 nucleotides in length of the antisense strand comprises in the 5' to 3' direction: CU, GG, or GA, the 5' end of the antisense strand in the siRNA forms a blunt end with the 3' end of the sense strand, and the antisense strand comprises phosphorothioate groups at the following internucleotide linkages: between positions 1 and 2 numbered from the 5' end, between positions 2 and 3 numbered from the 5' end, between positions 1 and 2 numbered from the 3' end, and between positions 2 and 3 numbered from the 3' end, and the nucleotide at position 1 of the antisense strand in the 5' end to 3' end direction is U or A, the nucleotide at position 2 of the antisense strand in the 5' end to 3' end direction is not U, the nucleotides at positions 2, 6, 7, 14, 16, and 22 of the antisense strand in the 5' end to 3' end direction are 2'-fluoro modified nucleotides, and the remainder of the nucleotides of the antisense strand modified with a 2'-methoxy group at the 2'- position of the ribose sugar moiety.
41. A siRNA for inhibiting the target gene expression, the siRNA comprises a sense strand and an antisense strand, wherein the sense strand is 21 continuous nucleotides in length, the antisense strand is 23 continuous nucleotides in length, the sense strand and the antisense strand comprise one or more of a 2'-methoxy modified nucleotide, a 2'-fluoro modified nucleotide, and a 5'-VP modified nucleotide, and comprise one or more phosphorothioate groups located between two adjacent nucleotides; at least one nucleotide at positions 19, 20, or 21 of the antisense strand in the 5' end to 3' end direction is mismatched to the target gene, the nucleotide of the antisense strand is identical to the nucleotide of the target gene at the mismatch position, and the nucleotide of the antisense strand and the nucleotide of the sense strand are complementary paired at the mismatch position, an overhang of 2 nucleotides in length of the antisense strand comprises in the 5' to 3' direction: CU, GG, or GA, the 5' end of the antisense strand in the siRNA forms a blunt end with the 3' end of the sense strand, and the antisense strand comprises phosphorothioate groups at the following internucleotide linkages: between positions 1 and 2 numbered from the 5' end, between positions 2 and 3 numbered from the 5' end, between positions 1 and 2 numbered from the 3' end, and between positions 2 and 3 numbered from the 3' end, and the nucleotide at position 1 of the antisense strand in the 5' end to 3' end direction is U or A, the nucleotide at position 2 of the antisense strand in the 5' end to 3' end direction is not U, the nucleotides at positions 2, 6, 7, 14, 16, and 22 of the antisense strand in the 5' end to 3' end direction are 2'-fluoro modified nucleotides, the remainder of the nucleotides of the antisense strand modified with a 2'-methoxy group at the 2'- position of the ribose sugar moiety, and the nucleotide at position 1 of the antisense strand in the 5' end to 3' end direction further has a 5'-VP modification, preferably a 5'-(E)-VP modification.
42. A siRNA for inhibiting the target gene expression, the siRNA comprises a sense strand and an antisense strand, wherein the sense strand is 21 continuous nucleotides in length, the antisense strand is 23 continuous nucleotides in length, one or more phosphorothioate groups of the sense strand and the antisense strand located between two adjacent nucleotides; at least one nucleotide at positions 19, 20, or 21 of the antisense strand in the 5' end to 3' end direction is mismatched to the target gene, the nucleotide of the antisense strand is identical to the nucleotide of the target gene at the mismatch position, and the nucleotide of the antisense strand and the nucleotide of the sense strand are complementary paired at the mismatch position, an overhang of 2 nucleotides in length of the antisense strand comprises in the 5' to 3' direction: CU, GG, or GA, the 5' end of the antisense strand in the siRNA forms a blunt end with the 3' end of the sense strand, and the antisense strand comprises phosphorothioate groups at the following internucleotide linkages: between positions 1 and 2 numbered from the 5' end, between positions 2 and 3 numbered from the 5' end, between positions 1 and 2 numbered from the 3' end, and between positions 2 and 3 numbered from the 3' end, and the nucleotide at position 1 of the antisense strand in the 5' end to 3' end direction is U or A, the nucleotide at position 2 of the antisense strand in the 5' end to 3' end direction is not U, the nucleotides at positions 2, 6, 7, 14, 16, and 22 of the antisense strand in the 5' end to 3' end direction are 2'-fluoro modified nucleotides, the remainder of the nucleotides of the antisense strand modified with a 2'-methoxy group at the 2'- position of the ribose sugar moiety, and the nucleotide at position 1 of the antisense strand in the 5' end to 3' end direction further has a 5'-VP modification, preferably a 5'-(*E*)-VP modification, the sense strand comprises no more than six 2'-fluoro modified nucleotides; and the remainder of the nucleotides of the sense strand are all 2'-methoxy modified nucleotides.
43. A siRNA for inhibiting the target gene expression, the siRNA comprises a sense strand and an antisense strand, wherein the sense strand is 21 continuous nucleotides in length, the antisense strand is 23 continuous nucleotides in length, one or more phosphorothioate groups of the sense strand and the antisense strand located between two adjacent nucleotides; at least one nucleotide at positions 19, 20, or 21 of the antisense strand in the 5' end to 3' end direction is mismatched to the target gene, the nucleotide of the antisense strand is identical to the nucleotide of the target gene at the mismatch position, and the nucleotide of the antisense strand and the nucleotide of the sense strand are complementary paired at the mismatch position, an overhang of 2 nucleotides in length of the antisense strand comprises in the 5' to 3' direction: CU, GG, or GA, the 5' end of the antisense strand in the siRNA forms a blunt end with the 3' end of the sense strand, and the antisense strand comprises phosphorothioate groups at the following internucleotide linkages: between positions 1 and 2 numbered from the 5' end, between positions 2 and 3 numbered from the 5' end, between positions 1 and 2 numbered from the 3' end, and between positions 2 and 3 numbered from the 3' end, and the nucleotide at position 1 of the antisense strand in the 5' end to 3' end direction is U or A, the nucleotide at position 2 of the antisense strand in the 5' end to 3' end direction is not U, the nucleotides at positions 2, 6, 7, 14, 16, and 22 of the antisense strand in the 5' end to 3' end direction are 2'-fluoro modified nucleotides, the remainder of the nucleotides of the antisense strand modified with a 2'-methoxy group at the 2'- position of the ribose sugar moiety, and the nucleotide at position 1 of the antisense strand in the 5' end to 3' end direction further has a 5'-VP modification, preferably a 5'-(*E*)-VP modification, the sense strand comprises five to six 2'-fluoro modified nucleotides; and the remainder of the nucleotides of the sense strand are all 2'-methoxy modified nucleotides.
44. A siRNA for inhibiting the target gene expression, the siRNA comprises a sense strand and an antisense strand, wherein the sense strand is 21 continuous nucleotides in length, the antisense strand is 23 continuous nucleotides in length, one or more phosphorothioate groups of the sense strand and the antisense strand located between two adjacent nucleotides; at least one nucleotide at positions 19, 20, or 21 of the antisense strand in the 5' end to 3' end direction is mismatched to the target gene, the nucleotide of the antisense strand is identical to the nucleotide of the target gene at the mismatch position, and the nucleotide of the antisense strand and the nucleotide of the sense strand are complementary paired at the mismatch position, an overhang of 2 nucleotides in length of the antisense strand comprises in the 5' to 3' direction: CU, GG, or GA, the 5' end of the antisense strand in the siRNA forms a blunt end with the 3' end of the sense strand, and the antisense strand comprises phosphorothioate groups at the following internucleotide linkages: between positions 1 and 2 numbered from the 5' end, between positions 2 and 3 numbered from the 5' end, between positions 1 and 2 numbered from the 3' end, and between positions 2 and 3 numbered from the 3' end, and the nucleotide at position 1 of the antisense strand in the 5' end to 3' end direction is U or A, the nucleotide at position 2 of the antisense strand in the 5' end to 3' end direction is not U, the nucleotides at positions 2, 6, 7, 14, 16, and 22 of the antisense strand in the 5' end to 3' end direction are 2'-fluoro modified nucleotides, the remainder of the nucleotides of the antisense strand modified with a 2'-methoxy group at the 2'- position of the ribose sugar moiety, and the nucleotide at position 1 of the antisense strand in the 5' end to 3' end direction further has a 5'-VP modification, preferably a 5'-(*E*)-VP modification, the nucleotides at positions 7, 9, 10, 11, and 13 of the sense strand in the 5' to 3' direction are 2'-fluoro modified nucleotides, and the remainder of the nucleotides of the sense strand are 2'-methoxy modified nucleotides.
45. A siRNA for inhibiting the target gene expression, the siRNA comprises a sense strand and an antisense strand, wherein the sense strand is 21 continuous nucleotides in length, the antisense strand is 23 continuous nucleotides in length, the sense strand and the antisense strand comprise one or more of a 2'-methoxy modified nucleotide, a 2'-fluoro modified nucleotide, and a 5'-VP modified nucleotide; at least one nucleotide at positions 19, 20, or 21 of the antisense strand in the 5' end to 3' end direction is mismatched to the target gene, the nucleotide of the antisense strand is identical to the nucleotide of the target gene at the mismatch position, and the nucleotide of the antisense strand and the nucleotide of the sense strand are complementary paired at the mismatch position, an overhang of 2 nucleotides in length of the antisense strand comprises in the 5' to 3' direction: CU, GG, or GA, the 5' end of the antisense strand in the siRNA forms a blunt end with the 3' end of the sense strand, and the antisense strand comprises phosphorothioate groups at the following internucleotide linkages: between positions 1 and 2 numbered from the 5' end, between positions 2 and 3 numbered from the 5' end, between positions 1 and 2 numbered from the 3' end, and between positions 2 and 3 numbered from the 3' end, and the nucleotide at position 1 of the antisense strand in the 5' end to 3' end direction is U or A, the nucleotide at position 2 of the antisense strand in the 5' end to 3' end direction is not U, the nucleotides at positions 2, 4, 6, 14, and 16 of the antisense strand in the 5' end to 3' end direction are 2'-fluoro modified nucleotides, and the remainder of the nucleotides of the antisense strand modified with a 2'-methoxy group at the 2'- position of the ribose sugar moiety, the nucleotides at positions 7, 9, 10, 11, and 13 of the 5' to 3' direction of the sense strand are 2'-fluoro modified nucleotides, and the remainder of the nucleotides of the sense strand are all 2'-methoxy modified nucleotides, the sense strand comprises phosphorothioate groups at one or more of the following internucleotide linkages: between positions 1 and 2 numbered from the 5' end, between positions 2 and 3 numbered from the 5' end, between positions 1 and 2 numbered from the 3' end, and between positions 2 and 3 numbered from the 3' end.
46. A siRNA for inhibiting the target gene expression, the siRNA comprises a sense strand and an antisense strand, wherein the sense strand is 21 continuous nucleotides in length, the antisense strand is 23 continuous nucleotides in length, the sense strand and the antisense strand comprise one or more of a 2'-methoxy modified nucleotide, a 2'-fluoro modified nucleotide, and a 5'-VP modified nucleotide; at least one nucleotide at positions 19, 20, or 21 of the antisense strand in the 5' end to 3' end direction is mismatched to the target gene, the nucleotide of the antisense strand is identical to the nucleotide of the target gene at the mismatch position, and the nucleotide of the antisense strand and the nucleotide of the sense strand are complementary paired at the mismatch position,, an overhang of 2 nucleotides in length of the antisense strand comprises in the 5' to 3' direction: CU, GG, or GA, the 5' end of the antisense strand in the siRNA forms a blunt end with the 3' end of the sense strand, and the antisense strand comprises phosphorothioate groups at the following internucleotide linkages: between positions 1 and 2 numbered from the 5' end, between positions 2 and 3 numbered from the 5' end, between positions 1 and 2 numbered from the 3' end, and between positions 2 and 3 numbered from the 3' end, and the nucleotide at position 1 of the antisense strand in the 5' end to 3' end direction is U or A, the nucleotide at position 2 of the antisense strand in the 5' end to 3' end direction is not U, the nucleotides at positions 2, 4, 6, 14, and 16 of the antisense strand in the 5' end to 3' end direction are 2'-fluoro modified nucleotides, and the remainder of the nucleotides of the antisense strand modified with a 2'-methoxy group at the 2'- position of the ribose sugar moiety, the nucleotides at positions 7, 9, 10, 11, and 13 of the 5' to 3' direction of the sense strand are 2'-fluoro modified nucleotides, and the remainder of the nucleotides of the sense strand are all 2'-methoxy modified nucleotides, and the sense strand comprises phosphorothioate groups at the following internucleotide linkages: between positions 1 and 2 numbered from the 5' end, between positions 2 and 3 numbered from the 5' end.
47. A siRNA for inhibiting the target gene expression, the siRNA comprises a sense strand and an antisense strand, wherein the sense strand is 21 continuous nucleotides in length, the antisense strand is 23 continuous nucleotides in length, the sense strand and the antisense strand comprise one or more of a 2'-methoxy modified nucleotide, a 2'-fluoro modified nucleotide, and a 5'-VP modified nucleotide; at least one nucleotide at positions 19, 20, or 21 of the antisense strand in the 5' end to 3' end direction is mismatched to the target gene, the nucleotide of the antisense strand is identical to the nucleotide of the target gene at the mismatch position, and the nucleotide of the antisense strand and the nucleotide of the sense strand are complementary paired at the mismatch position, an overhang of 2 nucleotides in length of the antisense strand comprises in the 5' to 3' direction: CU, GG, or GA, the 5' end of the antisense strand in the siRNA forms a blunt end with the 3' end of the sense strand, and the antisense strand comprises phosphorothioate groups at the following internucleotide linkages: between positions 1 and 2 numbered from the 5' end, between positions 2 and 3 numbered from the 5' end, between positions 1 and 2 numbered from the 3' end, and between positions 2 and 3 numbered from the 3' end, and the nucleotide at position 1 of the antisense strand in the 5' end to 3' end direction is U or A, the nucleotide at position 2 of the antisense strand in the 5' end to 3' end direction is not U, the nucleotides at positions 2, 4, 6, 14, and 16 of the antisense strand in the 5' end to 3' end direction are 2'-fluoro modified nucleotides, and the remainder of the nucleotides of the antisense strand modified with a 2'-methoxy group at the 2'- position of the ribose sugar moiety, the nucleotides at positions 7, 9, 10, 11, and 13 of the 5' to 3' direction of the sense strand are 2'-fluoro modified nucleotides, and the remainder of the nucleotides of the sense strand are all 2'-methoxy modified nucleotides, and the sense strand comprises phosphorothioate groups at the following internucleotide linkages: between positions 1 and 2 numbered from the 5' end, between positions 2 and 3 numbered from the 5' end, between positions 1 and 2 numbered from the 3' end, and between positions 2 and 3 numbered from the 3' end.
48. A siRNA for inhibiting the target gene expression, the siRNA comprises a sense strand and an antisense strand, wherein the sense strand is 21 continuous nucleotides in length, the antisense strand is 23 continuous nucleotides in length, the sense strand and the antisense strand comprise one or more of a 2'-methoxy modified nucleotide, a 2'-fluoro modified nucleotide, and a 5'-VP modified nucleotide; at least one nucleotide at positions 19, 20, or 21 of the antisense strand in the 5' end to 3' end direction is mismatched to the target gene, the nucleotide of the antisense strand is identical to the nucleotide of the target gene at the mismatch position, and the nucleotide of the antisense strand and the nucleotide of the sense strand are complementary paired at the mismatch position, an overhang of 2 nucleotides in length of the antisense strand comprises in the 5' to 3' direction: CU, GG, or GA, the 5' end of the antisense strand in the siRNA forms a blunt end with the 3' end of the sense strand, and the antisense strand comprises phosphorothioate groups at the following internucleotide linkages: between positions 1 and 2 numbered from the 5' end, between positions 2 and 3 numbered from the 5' end, between positions 1 and 2 numbered from the 3' end, and between positions 2 and 3 numbered from the 3' end, and the nucleotide at position 1 of the antisense strand in the 5' end to 3' end direction is U or A, the nucleotide at position 2 of the antisense strand in the 5' end to 3' end direction is not U, the nucleotides at positions 2, 6, 10, 14, and 16 of the antisense strand in the 5' end to 3' end direction are 2'-fluoro modified nucleotides, and the remainder of the nucleotides of the antisense strand modified with a 2'-methoxy group at the 2'- position of the ribose sugar moiety, the nucleotides at positions 3, 7, 9, 10, and 11 of the 5' to 3' direction of the sense strand are 2'-fluoro modified nucleotides, and the remainder of the nucleotides of the sense strand are all 2'-methoxy modified nucleotides, and the sense strand comprises phosphorothioate groups at the following internucleotide linkages: between positions 1 and 2 numbered from the 5' end, between positions 2 and 3 numbered from the 5' end.
49. A siRNA for inhibiting the target gene expression, the siRNA comprises a sense strand and an antisense strand, wherein the sense strand is 21 continuous nucleotides in length, the antisense strand is 23 continuous nucleotides in length, the sense strand and the antisense strand comprise one or more of a 2'-methoxy modified nucleotide, a 2'-fluoro modified nucleotide, and a 5'-VP modified nucleotide; at least one nucleotide at positions 19, 20, or 21 of the antisense strand in the 5' end to 3' end direction is mismatched to the target gene, the nucleotide of the antisense strand is identical to the nucleotide of the target gene at the mismatch position, and the nucleotide of the antisense strand and the nucleotide of the sense strand are complementary paired at the mismatch position, an overhang of 2 nucleotides in length of the antisense strand comprises in the 5' to 3' direction: CU, GG, or GA, the 5' end of the antisense strand in the siRNA forms a blunt end with the 3' end of the sense strand, and the antisense strand comprises phosphorothioate groups at the following internucleotide linkages: between positions 1 and 2 numbered from the 5' end, between positions 2 and 3 numbered from the 5' end, between positions 1 and 2 numbered from the 3' end, and between positions 2 and 3 numbered from the 3' end, and the nucleotide at position 1 of the antisense strand in the 5' end to 3' end direction is U or A, the nucleotide at position 2 of the antisense strand in the 5' end to 3' end direction is not U, the nucleotides at positions 2, 6, 14, 16, and 22 of the antisense strand in the 5' end to 3' end direction are 2'-fluoro modified nucleotides, and the remainder of the nucleotides of the antisense strand modified with a 2'-methoxy group at the 2'- position of the ribose sugar moiety, the nucleotides at positions 7, 9, 10, 11, and 13 of the 5' to 3' direction of the sense strand are 2'-fluoro modified nucleotides, and the remainder of the nucleotides of the sense strand are all 2'-methoxy modified nucleotides, and the sense strand comprises phosphorothioate groups at the following internucleotide linkages: between positions 1 and 2 numbered from the 5' end, between positions 2 and 3 numbered from the 5' end.
50. A siRNA for inhibiting the target gene expression, the siRNA comprises a sense strand and an antisense strand, wherein the sense strand is 21 continuous nucleotides in length, the antisense strand is 23 continuous nucleotides in length, the sense strand and the antisense strand comprise one or more of a 2'-methoxy modified nucleotide, a 2'-fluoro modified nucleotide, and a 5'-VP modified nucleotide; at least one nucleotide at positions 19, 20, or 21 of the antisense strand in the 5' end to 3' end direction is mismatched to the target gene, the nucleotide of the antisense strand is identical to the nucleotide of the target gene at the mismatch position, and the nucleotide of the antisense strand and the nucleotide of the sense strand are complementary paired at the mismatch position, an overhang of 2 nucleotides in length of the antisense strand comprises in the 5' to 3' direction: CU, GG, or GA, the 5' end of the antisense strand in the siRNA forms a blunt end with the 3' end of the sense strand, and the antisense strand comprises phosphorothioate groups at the following internucleotide linkages: between positions 1 and 2 numbered from the 5' end, between positions 2 and 3 numbered from the 5' end, between positions 1 and 2 numbered from the 3' end, and between positions 2 and 3 numbered from the 3' end, and the nucleotide at position 1 of the antisense strand in the 5' end to 3' end direction is U or A, the nucleotide at position 2 of the antisense strand in the 5' end to 3' end direction is not U, the nucleotides at positions 2, 6, 14, 16 and 22 of the antisense strand in the 5' end to 3' end direction are 2'-fluoro modified nucleotides, and the remainder of the nucleotides of the antisense strand modified with a 2'-methoxy group at the 2'- position of the ribose sugar moiety, the nucleotides at positions 7, 9, 10, 11, 13, and 17 of the 5' to 3' direction of the sense strand are 2'-fluoro modified nucleotides, and the remainder of the nucleotides of the sense strand are all 2'-methoxy modified nucleotides, and the sense strand comprises phosphorothioate groups at the following internucleotide linkages: between positions 1 and 2 numbered from the 5' end, between positions 2 and 3 numbered from the 5' end.
51. A siRNA for inhibiting the target gene expression, the siRNA comprises a sense strand and an antisense strand, wherein the sense strand is 21 continuous nucleotides in length, the antisense strand is 23 continuous nucleotides in length, the sense strand and the antisense strand comprise one or more of a 2'-methoxy modified nucleotide, a 2'-fluoro modified nucleotide, and a 5'-VP modified nucleotide; at least one nucleotide at positions 19, 20, or 21 of the antisense strand in the 5' end to 3' end direction is mismatched to the target gene, the nucleotide of the antisense strand is identical to the nucleotide of the target gene at the mismatch position, and the nucleotide of the antisense strand and the nucleotide of the sense strand are complementary paired at the mismatch position, an overhang of 2 nucleotides in length of the antisense strand comprises in the 5' to 3' direction: CU, GG, or GA, the 5' end of the antisense strand in the siRNA forms a blunt end with the 3' end of the sense strand, and the antisense strand comprises phosphorothioate groups at the following internucleotide linkages: between positions 1 and 2 numbered from the 5' end, between positions 2 and 3 numbered from the 5' end, between positions 1 and 2 numbered from the 3' end, and between positions 2 and 3 numbered from the 3' end, and the nucleotide at position 1 of the antisense strand in the 5' end to 3' end direction is U or A, the nucleotide at position 2 of the antisense strand in the 5' end to 3' end direction is not U, the nucleotides at positions 2, 6, 14, 16, and 22 of the antisense strand in the 5' end to 3' end direction are 2'-fluoro modified nucleotides, and the remainder of the nucleotides of the antisense strand modified with a 2'-methoxy group at the 2'- position of the ribose sugar moiety, the nucleotide at position 1 of the antisense strand in the 5' end to 3' end direction further has a 5'-VP modification, preferably a 5'-(E)-VP modification, the nucleotides at positions 7, 9, 10, 11, and 13 of the 5' to 3' direction of the sense strand are 2'-fluoro modified nucleotides, and the remainder of the nucleotides of the sense strand are all 2'-methoxy modified nucleotides, and the sense strand comprises phosphorothioate groups at the following internucleotide linkages: between positions 1 and 2 numbered from the 5' end, between positions 2 and 3 numbered from the 5' end.
52. A siRNA for inhibiting the target gene expression, the siRNA comprises a sense strand and an antisense strand, wherein the sense strand is 21 continuous nucleotides in length, the antisense strand is 23 continuous nucleotides in length, at least one nucleotide at positions 19, 20, or 21 of the antisense strand in the 5' end to 3' end direction is mismatched to the target gene, the nucleotide of the antisense strand is identical to the nucleotide of the target gene at the mismatch position, and the nucleotide of the antisense strand and the nucleotide of the sense strand are complementary paired at the mismatch position, an overhang of 2 nucleotides in length of the antisense strand comprises in the 5' to 3' direction: CU, GG, or GA, the 5' end of the antisense strand in the siRNA forms a blunt end with the 3' end of the sense strand, and the antisense strand comprises phosphorothioate groups at the following internucleotide linkages: between positions 1 and 2 numbered from the 5' end, between positions 2 and 3 numbered from the 5' end, between positions 1 and 2 numbered from the 3' end, and between positions 2 and 3 numbered from the 3' end, and the nucleotide at position 1 of the antisense strand in the 5' to 3' direction is U or A, the nucleotide at position 2 of the antisense strand in the 5' end to 3' end direction is not U, the nucleotides at positions 2, 6, 7, 14, 16, and 22 of the antisense strand in the 5' end to 3' end direction are 2'-fluoro modified nucleotides, the remainder of the nucleotides of the antisense strand modified with a 2'-methoxy group at the 2'-position of the ribose sugar moiety, the nucleotide at position 1 of the antisense strand in the 5' end to 3' end direction further has a 5'-VP modification, preferably a 5'-(E)-VP modification, the nucleotides at positions 7, 9, 10, 11, and 13 of the 5' to 3' direction of the sense strand are 2'-fluoro modified nucleotides, and the remainder of the nucleotides of the sense strand are all 2'-methoxy modified nucleotides, and the sense strand comprises phosphorothioate groups at the following internucleotide linkages: between positions 1 and 2 numbered from the 5' end, between positions 2 and 3 numbered from the 5' end.

In some embodiments of the present disclosure, "target gene" refers to a target mRNA. In the context of the present disclosure, a "target mRNA" refers to an mRNA corresponding to a gene that is aberrantly expressed in a cell.

In some embodiments of the present disclosure, the target mRNA specifically refers to an mRNA corresponding to an overexpressed gene.

In some embodiments of the present disclosure, the target mRNA may be an mRNA corresponding to a gene selected from ApoB, ApoC, ANGPLT3, PSCK9, SCD1, TIMP-1, CoL11A1, FVII, STAT3, p53, HBV, HCV, FXI, FXII, KNG, PNP, XO, PKK, PLG, C9/SARS, SARS-CoV-2, ACE-2, or AGT, etc.

In some embodiments of the present disclosure, the target mRNA may be an mRNA corresponding to a gene selected from IL-4, IL-4Rα, IL-6, IL-8, IL-10, IL-13, C3, C5, AR, INHBE, GPR75, KHK, TNF-α, RAGE, CIDEB, HSD17B13, SCAP, AAT, CTSC, CFB, Lp(a), ALAS1, TGF-β, or TTR, etc.

In some embodiments of the present disclosure, the target mRNA may be an mRNA corresponding to an ANGPLT3, AGT, or RAGE gene.

In some embodiments of the present disclosure, the target mRNA may be an mRNA corresponding to an ANGPLT3 or RAGE gene.

In some embodiments of the present disclosure, the target mRNA may be an mRNA corresponding to an ANGPLT3 or AGT gene.

In some embodiments of the present disclosure, the target mRNA is an mRNA corresponding to a RAGE gene.

In some embodiments of the present disclosure, the target gene is the RAGE gene.

In some embodiments of the present disclosure, the antisense strand of the siRNA is selected from any one of the antisense strands shown in Table 1.

In some embodiments of the present disclosure, the antisense strand of the siRNA is selected from any one of the antisense strands shown in Table 1, and the sense strand of the siRNA is selected from any one of the sense strands shown in Table 1. It will be appreciated by those skilled in the art that the ligands contained in the partial sense strands shown in Table 1 are obtained by conjugation in the specific examples and that the duplex siRNA prior to conjugation of the ligands should also be considered to be within the scope of the present disclosure.

It will be appreciated by those skilled in the art that siRNA, according to the present disclosure, may be obtained by conventional methods of siRNA preparation in the art, such as solid-phase synthesis or liquid-phase synthesis. Among them, both solid-phase synthesis and liquid-phase synthesis have been commercially subscribed to services. It is also clear to the person skilled in the art that modified nucleotide groups may be introduced into the siRNA described in the present disclosure by using nucleotide monomers with corresponding modifications. Methods for preparing nucleotide monomers with corresponding modifications are well known to those skilled in the art, and commercially available monomers are commercially available.

### II. siRNA conjugates of the present disclosure

The siRNA conjugates of the present disclosure result from the conjugation of a siRNA of the present disclosure with a pharmaceutically acceptable conjugating molecule (including one or more conjugating molecules, e. g., one, two, three, or four) including a pharmaceutically acceptable targeting ligand and optionally a linker. In some embodiments of the present disclosure, the siRNA is covalently conjugated to the conjugating molecule. In order to reduce the possible effect of conjugation on the activity of siRNA, the site of conjugation of the siRNA to the conjugating molecule may be at the 3' or 5' end of the sense strand of the siRNA, or at the 5' end of the antisense strand. In some embodiments, the site of conjugation of the siRNA to the conjugated molecule may also be within the internal sequence of the siRNA.

The pharmaceutically acceptable targeting ligand may be a targeting ligand conventionally used in the art of siRNA administration, such as, but not limited to, one or more of the following targeting ligands or derivatives thereof: lipophilic molecules such as cholesterol, bile acids, vitamins (e. g. Vitamin E), lipid molecules of different chain length (e. g. Phospholipids); a polymer such as polyethylene glycol; a polypeptide, e. g. a cell-penetrating peptide; an aptamer; an antibody; quantum dots; saccharides, such as lactose, polyglactose, mannose, galactose, N-acetylgalactosamine (GalNAc) or derivatives thereof (GalNAc (L96), also referred to as "L96"), CMM (Chemically Modified Mannose); folate; or a receptor ligand expressed by hepatocytes, for example, asialoglycoproteins, asialo sugar residues, lipoproteins (e. g. high density lipoproteins, low density lipoproteins, etc.), glucagon, neurotransmitters (e. g. epinephrine), growth factors, transferrin, etc.

In some embodiments of the present disclosure, the targeting ligand is N-acetylgalactosamine or CMM. In some embodiments of the present disclosure, the targeting ligand is N-acetylgalactosamine. In some embodiments of the present disclosure, the Targeting Ligand is the N-acetylgalactosamine derivative GalNAc (L96).

In some embodiments of the present disclosure, the targeting ligand is directly attached to the 3' end of the sense strand of the siRNA. In some embodiments of the present disclosure, the targeting ligand is linked to the 3' end of the sense strand of the siRNA via a linker. In some embodiments of the present disclosure, the targeting ligand is linked to the 3' end of the sense strand of the siRNA via a linker through a phosphate linkage or a phosphorothioate group.

In some embodiments of the present disclosure, the targeting ligand is directly attached to the 5' end of the sense strand of the siRNA. In some embodiments of the present disclosure, the targeting ligand is linked to the 5' end of the sense strand of the siRNA via a linker. In some embodiments of the present disclosure, the targeting ligand is linked to the 5' end of the sense strand of the siRNA via a linker through a phosphate linkage or a phosphorothioate group.

In some embodiments of the present disclosure, the linker-targeting ligand moiety has the structure: 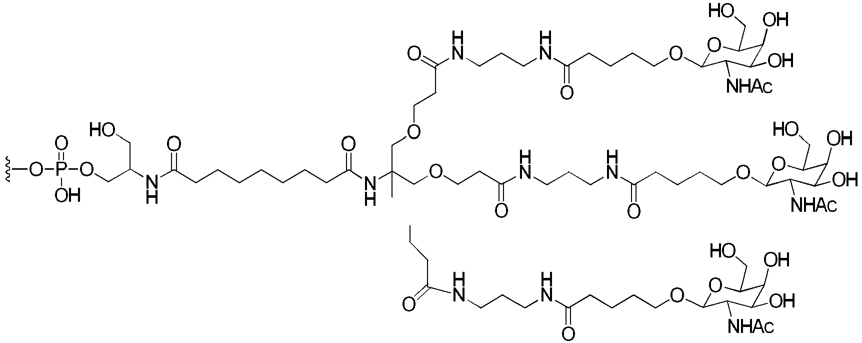 or 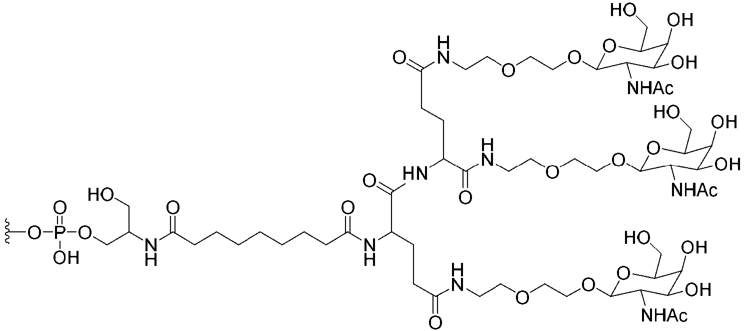 or 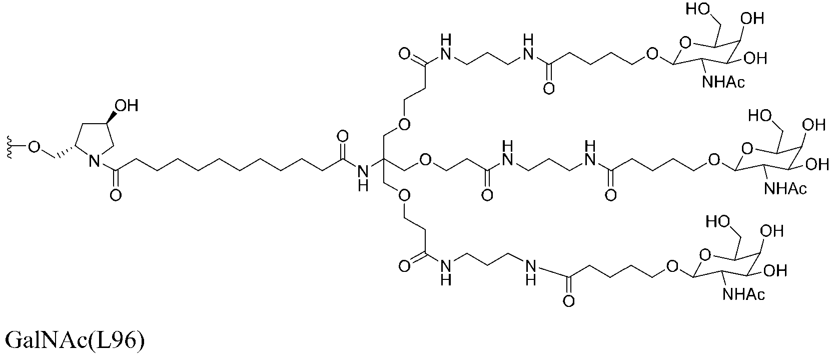 or

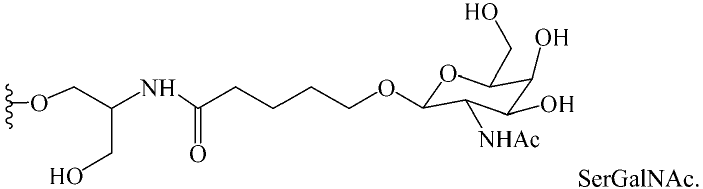

Optionally, the linker-targeting ligand moiety is attached to the 3' end of the sense strand of the siRNA as a phosphate or phosphorothioate.

In some embodiments of the present disclosure, the conjugating molecule is covalently conjugated to the 3' end of the sense strand of siRNA in the manner shown below: 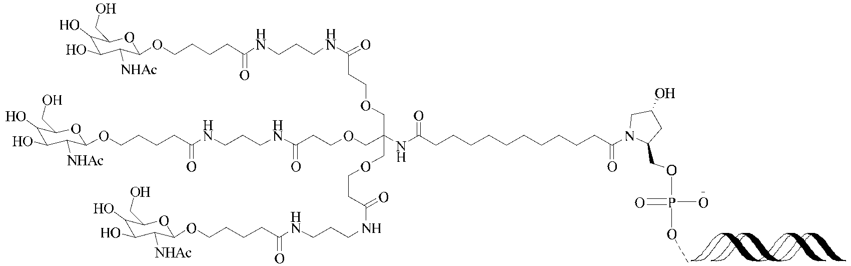 wherein the conjugating molecule described herein is covalently conjugated to the 3' end of the sense strand of the siRNA shown above

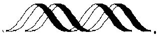

In some embodiments of the present disclosure, there is provided any one of the following siRNA conjugates:
1. A siRNA conjugate is obtained by conjugating a siRNA or a modified siRNA as described in any of the present disclosure to a pharmaceutically acceptable conjugating molecule.
2. A siRNA conjugate results from the conjugation of a siRNA or a modified siRNA as described in any one of the present disclosures with a pharmaceutically acceptable conjugating molecule including a pharmaceutically acceptable targeting ligand and optionally a linker.
3. A siRNA conjugate is obtained by conjugating a siRNA or a modified siRNA as described in any of the present disclosure to a pharmaceutically acceptable conjugating molecule including a pharmaceutically acceptable targeting ligand, which is GalNAc or a derivative thereof, and optionally a linker.
4. A siRNA conjugate is obtained by conjugating a siRNA or modified siRNA as described in any of the present disclosure to a pharmaceutically acceptable conjugating molecule including a pharmaceutically acceptable targeting ligand and optionally a linker, the targeting ligand being GalNAc (L96).
5. A siRNA conjugate is obtained by conjugating a siRNA or a modified siRNA as described in any of the present disclosure to a pharmaceutically acceptable conjugating molecule including a pharmaceutically acceptable targeting ligand, which is GalNAc (L96), and optionally a linker, linked through the optional linker as a phosphate or phosphorothioate to the 3' end or the 5' end of the sense strand of the siRNA.
6. A siRNA conjugate is obtained by conjugating a siRNA or a modified siRNA of any of the present disclosure to a pharmaceutically acceptable conjugating molecule including a pharmaceutically acceptable targeting ligand and optionally a linker, the targeting ligand being GalNAc (L96), the conjugating molecule being covalently conjugated to the 3' end of the sense strand of the siRNA in the manner shown below: 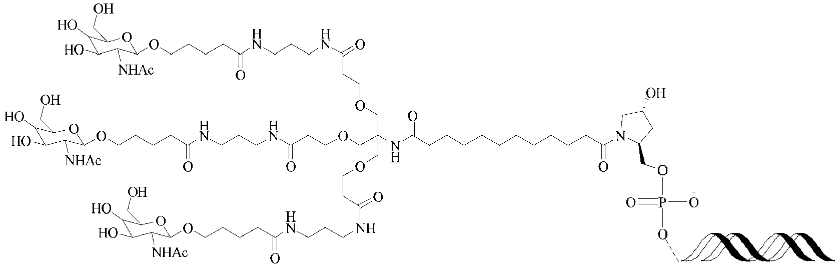 wherein 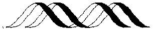 represents the siRNA.

### III. siRNA pharmaceutical compositions of the present disclosure

The present disclosure also provides a pharmaceutical composition comprising the siRNA, modified siRNA, or siRNA conjugate of the present disclosure as an active ingredient and a pharmaceutically acceptable carrier.

In some embodiments of the present disclosure, the pharmaceutical composition comprises one siRNA, modified siRNA, or siRNA conjugate as described in any of the herein. In other embodiments of the present disclosure, the pharmaceutical composition comprises at least two siRNA, modified siRNA, or siRNA conjugates (such as, but not limited to, 2, 3, 4, 5, 6, 7, 8, 9, 10, or more) as described in any of the herein as active ingredients. Optionally, each of the at least two siRNA, modified siRNA, or siRNA conjugates as described in any of the herein targets a different target sequence in a target gene, whereby simultaneous action against different target sequences in a target gene can be expected to bring about a synergistic effect.

As used herein, the term "different target sequences" means that there is no overlap between the target sequences, or that there are less than 5 continuous nucleotides of overlap between the target sequences (e. g., 4, 3, 2, 1, 0 continuous nucleotides of overlap). In this case, at least two siRNA, modified siRNA, or siRNA conjugates as described in any of the embodiments herein may be present in any different ratio. Optionally, the at least two siRNA, modified siRNA, or siRNA conjugates as described in any of the herein may be present in a molar ratio of 1:100 to 100:1 to each other; optionally, the at least two siRNA, modified siRNA, or siRNA conjugates as described in any of the herein may be present in a molar ratio of from 1:10 to 10:1, from 1:5 to 5:1, or from 1:2 to 2:1 to each other. In some embodiments of the present disclosure, at least two siRNA, modified siRNA, or siRNA conjugates as described in any of the present disclosure are present in the same molar ratio.

In some embodiments of the present disclosure, the pharmaceutically acceptable carrier comprises, but is not limited to, magnetic nanoparticles (e.g., Fe₃O₄, Fe₂O₃), carbon nanotubes, mesoporous silicon, calcium phosphate nanoparticles, polyethylenimine (PEI), polyamidoamine dendrimer (PAMAM) dendrimer, poly(L-lysine) (PLL), chitosan, 1,2-dioleoyl-3-trimethylammonium-propane (DOTAP), poly (D&L-lactic-co-glycolic acid) copolymer (PLGA), poly(2-aminoethyl ethylene phosphate) (PPEEA), poly(2-dimethylaminoethyl methacrylate) (PDMAEMA), and derivatives thereof, alone or in combination.

In the pharmaceutical composition of the present disclosure, no particular limitation is imposed on the amount of the pharmaceutically acceptable carrier; generally, the weight ratio of the total amount of siRNA, modified siRNA, or siRNA conjugate to the pharmaceutically acceptable carrier in the pharmaceutical composition is 1: (1-500), preferably 1: (1-50).

In some embodiments of the present disclosure, the pharmaceutical composition may further comprise a cationic component to assist in the in vivo delivery of the drug. The cationic component may be, but is not limited to, a positively charged polypeptide or protein, a cationic lipid, a positively charged polymer, and the like. As the positively charged polypeptide or protein, oligoarginine, oligo-lysine, protamine, and the like may be cited. As the cationic lipid, it may be at least one cationic lipid selected from the group consisting of dimethyl dioctadecyl (DDAB), 1,2-dimyristoyl-3-trimethylammonium propane, 1,2-dioleoyl-3-trimethylammonium propane (DOTAP), 1,2-dioleoyl-3-trimethylammonium propane methylsulfate, 1,2-dipalmitoyl-3-trimethylammonium propane, 1,2-distearoyl-3-trimethylammonium propane, N-(1-(2,3-dioleoyloxy) propyl)-N, N, N-trimethylammonium chloride (DOTMA), dimyristoyloxypropyl dimethyl hydroxyethyl ammonium bromide (DMRIE), dioleoyloxypropyl dimethyl hydroxyethyl ammonium bromide (DORIE), dimethyl didodecyl ammonium bromide, N-(a-trimethylammonioacetyl)-didodecyl-D-glutamine hydrochloride, N-(a-trimethylammonioacetyl)-O,O'-bis-(1H,1H,2H,2H-perfluorodecyl)-L-glutamine hydrochloride, O,O'-didodecanoyl-N-(a-trimethylammonioacetyl) diethanolamine hydrochloride, methylallyl didodecyl ammonium bromide, N-{p-(w-trimethylammonio butyloxy)-benzoyl}-di(dodecyl)-L-glutamine hydrochloride, 9-(w-trimethylammonio butyl)-3,6-bis (dodecanoyl) carbazole bromide, dimethyldioctadecyl ammonium hydrochloride, N-w-trimethylammonio decanoyl-di(hexadecyl)-D-glutamine bromide, N-{p-(w-trimethylammonio hexyloxy)-benzoyl}-di(tetradecyl)-L-glutamine bromide, p-(w-trimethylammonio decyloxy)-p '-octyloxyazobenzene bromide salt (MC-1-0810), p-{w-(b-hydroxyethyl) dimethyl-ammonio-decyloxy}-p'-octyloxyazobenzene bromide salt (MC-3-0810), O, O', O"-tris (dodecanoyl)-N-(w-trimethyl-ammoniodecanoyl)-tris (hydroxymethyl) aminomethane bromide salt (TC-1-12), 1,2-dilauryl-glycerol-3-ethylphosphocholine, 1,2-dimyristoyl-glycerol-3-ethylphosphocholine, 1,2-dipalmitoyl-glycerol-3-ethylphosphocholine, 1,2-distearoyl-glycerol-3-ethylphosphocholine, 1,2-dioleoyl-glycerol-3-ethylphosphocholine, 1-palmitoyl-2-oleoyl-glycerol-3-ethylphosphocholine, at least one cationic lipid of N, N-dihydroxyethyl-N-methyl-N-2-(cholesteryloxycarbonylamino) ethylammonium bromide (BHEM-Chol), (2,3-dioleoxypropyl) trimethylammonium chloride (DOTAP), and N-(1-(2,3-dioleoyloxy)-propyl)-N,N,N-trimethylammonium chloride. As the positively charged polymer, there may be at least one positively charged polymer selected from the group consisting of polyethylenimine, poly β-amino ester, and chitosan quaternary ammonium salt.

For the same purpose, the pharmaceutical composition may also comprise a non-cationic component. The non-cationic component may be, but is not limited to, a neutral membrane fusogenic lipid, an anionic lipid, an amphiphilic polymer, etc. Examples of the membrane fusogenic lipid include dioleoyl phosphatidyl ethanolamine, dioleoyl phosphatidyl choline, trans-phosphatidyl ethanolamine, 1,2-bis (10,12-tricosandioyl)-phosphoethanolamine, 1,2-di-elaidoyl phosphoethanolamine, 1,2-dihexadecyl phosphoethanolamine, 1,2-dihexanoyl phosphoethanolamine, 1,2-dilauroyl phosphoethanolamine, 1,2-dilinoleoyl phosphoethanolamine, 1,2-dimyristoyl phosphoethanolamine, 1,2-dioleoyl phosphoethanolamine, 1,2-dioleoyl phosphoethanolamine. 2-dipalmitoyl phosphoethanolamine, 1,2-dipalmitoyl phosphoethanolamine, 1,2-diphytanoyl phosphoethanolamine, 1,2-distearoyl phosphoethanolamine, 1-palmitoyl-2-oleoyl phosphoethanolamine, 1-palmitoyl-2-(10,12-tricosanedioyl) phosphoethanolamine, 1,2-dioleoyl phosphoethanolamine-N-hexanamide, 1,2-dipalmitoyl phosphoethanolamine-N-hexanamide, N,N-dimethyl-1,2-dioleoyl phosphoethanolamine, N,N-dimethyl-1,2-dioleoyl phosphoethanolamine, 2-dipalmitoyl phosphoethanolamine, N-dodecanoyl-1,2-dipalmitoyl phosphoethanolamine, N-dodecanoyl-1,2-dioleoyl phosphoethanolamine, 1,2-dioleoyl phosphoethanolamine-N-dodecylamine, 1,2-dipalmitoyl phosphoethanolamine-N-dodecylamine, 1,2-dioleoyl phosphoethanolamine-N-glutaryl, 1,2-dipalmitoyl phosphoethanolamine-N-glutaryl, 1,2-dioleoyl phosphoethanolamine-N-lactose, 1,2-dioleoyl phosphoethanolamine, 2-dioleoyl phosphoethanolamine-N-[4 (p-maleimidomethyl) cyclohexane-carboxylate], dipalmitoyl phosphoethanolamine-N-[4-(p-maleimidomethyl) cyclohexane-carboxylate], 1,2-dipalmitoyl phosphoethanolamine-N-[4-(p-maleimidophenyl) butanamide], 1,2-dioleoyl phosphoethanolamine-N-[4-(p-maleimidophenyl) butanoate], N-methyl-1, 2-dioleoyl phosphoethanolamine, N-methyl-dipalmitoyl phosphoethanolamine, 1,2-dioleoyl phosphoethanolamine-N-3-(2-pyridyldithio) propionate, 1,2-dipalmitoyl phosphoethanolamine-N-[3-(2-pyridyldithio) propionate], N-(succinyl)-1,2-dioleoyl phosphoethanolamine, N-(succinyl)-1,2-dipalmitoyl phosphoethanolamine, and the like.

In the pharmaceutical composition of the present disclosure, it is possible to further improve the transport and delivery efficiency of the pharmaceutical composition in a mammal by containing the above-mentioned membrane fusogenic lipid. As the amphiphilic polymer, polyethylene glycol-polylactic acid diblock copolymer, polyethylene glycol-polylactic acid triblock copolymer, polyethylene glycol-poly (lactic acid-glycolic acid) diblock copolymer, polyethylene glycol-poly (lactic acid-glycolic acid) triblock copolymer, polycaprolactone-polyphosphate diblock copolymer, polycaprolactone-polyphosphate triblock copolymer, polyethylene glycol-polycaprolactone diblock copolymer, polyethylene glycol-polycaprolactone triblock copolymer, and the like can be cited.

In some embodiments of the present disclosure, the pharmaceutical composition may further include a pharmaceutically acceptable additional excipient. The pharmaceutically acceptable other excipients may comprise at least one of a pH buffer, a protectant, and an osmotic pressure regulator. The buffer may be a Tris hydrochloride buffer at a pH of 7.5-8.5 and/or a phosphate-buffered saline at a pH of 5.5-8.5, preferably a phosphate-buffered saline at a pH of 5.5-8.5. The protectant may be at least one of inositol, sorbitol, sucrose, trehalose, mannose, maltose, lactose, and glucose. The protectant may be contained in an amount of 0.01 wt% to 30 wt%, based on the total weight of the pharmaceutical composition. The osmotic pressure regulators may be sodium chloride and/or potassium chloride. The osmotic pressure regulators are present in an amount such that the pharmaceutical composition has an osmolality of from 200 to 700 milliosmol/kg. The content of the osmotic pressure regulators may be easily determined by a person skilled in the art according to the desired osmotic pressure.

In some embodiments of the present disclosure, the pharmaceutical composition may be a liquid formulation, such as an injectable solution. It may also be a lyophilized powder for injection, which is mixed with a liquid excipient to prepare a liquid preparation when administration is carried out. The liquid formulation may be, but is not limited to, for subcutaneous, intramuscular, or intravenous administration, and may also be, but is not limited to, administration to the lung by nebulization, or administration to other organ tissues (e. g., liver) via the lung by nebulization.

Optionally, the pharmaceutical composition is for intravenous administration.

In some embodiments of the present disclosure, a pharmaceutical composition according to any one of the following is provided:
The pharmaceutical composition comprises the following components:
(a) an siRNA, a modified siRNA, or an siRNA conjugate as described in any of the embodiments herein;
(b) a pharmaceutically acceptable carrier.

### IV. The kit of the present disclosure

The kit of the present disclosure comprises a siRNA, a modified siRNA, a siRNA conjugate, or a pharmaceutical composition as described in the present disclosure. In the case where a plurality of siRNAs is present, each siRNA may be present alone or in a mixture of two or more species.

In addition to the siRNA, modified siRNA, siRNA conjugate, or pharmaceutical composition, components necessary or beneficial for achieving one or more particular applications of the present disclosure may also be comprised in the kit, such components comprising, but not limited to: (1) one or more components for achieving a desired cell transfection; (2) one or more components for effecting diagnosis, treatment or prevention of a particular disease, such as one or more additional therapeutic compounds or compositions, one or more diagnostic agents; (3) one or more buffers; (4) positive or negative control sample; (5) excipients, stabilizers or preservatives and the like.

In some embodiments of the present disclosure, the kit may further comprise instructions for use.

In some embodiments of the present disclosure, the siRNA, modified siRNA, siRNA conjugate, or pharmaceutical composition in the kit can be provided in any form, for example, a liquid form, a dried form, or a lyophilized form. In some embodiments of the present disclosure, the siRNA, modified siRNA, siRNA conjugate, or pharmaceutical composition in the kit is, in principle, pure and/or sterile.

In some embodiments of the present disclosure, there is provided a kit of any of the following:
The kit comprises a siRNA of any of the present disclosure, a modified siRNA of any of the present disclosure, a siRNA conjugate of any of the present disclosure, or a pharmaceutical composition of any of the present disclosure.

### V. Use of the siRNA, modified siRNA, siRNA conjugates, and pharmaceutical compositions of the disclosure

Administration of an siRNA, a modified siRNA, an siRNA conjugate, and/or a pharmaceutical composition of the present disclosure to a subject in need thereof effectively reduces target gene expression in the subject, thereby enabling the prevention and/or treatment of a disease in the subject, or use in preparing a pharmaceutical composition for preventing and/or treating a disease.

In some embodiments of the present disclosure, there is provided a method of preventing and/or treating one or more diseases or conditions associated with inhibiting the expression of at least one gene in ANGPTL3, AGT, or RAGE, the method including administering to a subject in need thereof the siRNA of any of the present disclosure, the modified siRNA of any of the present disclosure, or the siRNA conjugate of any of the present disclosure, or the pharmaceutical composition of any of the present disclosure, or the kit of any of the present disclosure.

As used herein, the term "administering/giving" refers to the placement of a siRNA into a subject by a method or route that at least partially locates the siRNA at a desired site to produce a desired effect. Routes of administration suitable for the methods of the present disclosure include topical administration and systemic administration. In general, topical administration results in the delivery of more siRNA to a particular site than the subject's entire body; systemic administration results in delivery of the siRNA to substantially the entire body of the subject. The subject may be administered by any suitable route known in the art, including, but not limited to: oral or parenteral routes, including intravenous administration, intramuscular administration, subcutaneous administration, transdermal administration, airway administration (aerosol), pulmonary administration, nasal administration, rectal administration, and topical administration (including buccal administration and sublingual administration). In addition, the frequency of administration can be one or more times daily, weekly, monthly, or annually.

The dosage of siRNA used in the present disclosure can be determined by a clinician, medical/pharmaceutical practitioner, etc., according to various parameters, in particular, the age, weight, sex, etc. of the subject. Toxicity and therapeutic efficacy can be determined by standard pharmaceutical procedures in cell cultures or animal experiments, e. g., for determining the LD₅₀ (the dose lethal to 50% of the population) and the ED₅₀ (the dose lethal to 50% of the population in a quantitative response and the dose lethal to 50% of the population in a qualitative response). The dose ratio between toxic and therapeutic effects is the therapeutic index, and it can be expressed as the ratio LD₅₀/ED₅₀. Preference is given to siRNA or siRNA conjugates or pharmaceutical compositions exhibiting a high therapeutic index. Ranges of dosage for human use can be derived based on data obtained from cell culture assays and animal experimental studies, and are within the ability of those skilled in the art.

When the siRNA, modified siRNA, siRNA conjugate or pharmaceutical composition of the present disclosure is administered, for example, to male or female C57BL/6J or C3H/HeNCrlVr mice aged 6-12 weeks and weighing 18-25 g, it may be in the range of 0.001-50 mg/kg body weight, preferably 0.01-10 mg/kg body weight, more preferably 0.05-5 mg/kg body weight, most preferably 0.1-3 mg/kg body weight, based on the amount of siRNA.

In some embodiments of the present disclosure, the siRNA, modified siRNA, siRNA conjugates, and/or pharmaceutical compositions of the present disclosure are used as the sole active ingredient for the prevention and/or treatment of a disease.

In some embodiments of the present disclosure, the siRNA, modified siRNA, siRNA conjugates, and/or pharmaceutical compositions of the present disclosure are used in conjunction with other active ingredients for the prevention and/or treatment of a disease.

It should be understood by those of ordinary skill in the art that the foregoing description of the present disclosure is exemplary and that the embodiments disclosed herein may be readily modified into other specific forms without departing from the technical spirit or essential characteristics of the present disclosure. It is therefore to be understood that the above-described embodiments are illustrative, and not restrictive, in all respects.

Unless specifically stated otherwise, the singular terms encompass the plural terms and the plural terms encompass the singular terms. The words "a" or "an" mean "at least one" or "at least one" unless specifically stated otherwise. The use of "or" means "and/or" unless stated otherwise.

All patents, patent applications, and other identified publications are expressly incorporated herein by reference for the purpose of description and disclosure. These publications are provided solely for their disclosure prior to the filing date of the present application. All statements as to the date of these documents or representation of the contents of these documents are based on the information available to the applicant and do not constitute any admission as to the correctness of the dates or contents of these documents. Moreover, any reference herein to such publications in any country does not constitute an admission that such publications are part of the common general knowledge in the art.

In the following detailed description, ANGPTL3, AGT, and RAGE are selected as exemplary target genes in the present disclosure to prepare corresponding siRNA duplexes, modified siRNA duplexes, and conjugates; the specific sequence information is set forth in Table 1 below:

**Table 1 Sequence information**

| Sense strand (5' to 3') | SEQ ID NO: | Antisense strand (5' to 3') | SEQ ID NO: |
|---|---|---|---|
| mA*mG*mCmCmAmAi2FAmAi2 FUi2FCi2FAmAi2FGmAmUmUm UmGmC*mU*mAGalNAc(L96) | 1 | mU*i2FA*mGi2FCmAi2FAmAmU mCmUmUmGmAi2FUmUi2FUmU mGmGmCmU*mC*mU | 2 |
| mU*mG*mCmCmAmAi2FAmAi2 FUi2FCi2FAmAi2FGmAmUmUm UmGmC*mU*mAGalNAc(L96) | 3 | mU*i2FA*mGi2FCmAi2FAmAmU mCmUmUmGmAi2FUmUi2FUmU mGmGmCmA*mC*mU | 4 |
| mU*mC*mCmCmAmAi2FAmAi2 FUi2FCi2FAmAi2FGmAmUmUm UmGmC*mU*mAGalNAc(L96) | 5 | mU*i2FA*mGi2FCmAi2FAmAmU mCmUmUmGmAi2FUmUi2FUmU mGmGmGmA*mC*mU | 6 |
| mU*mC*mGmCmAmAi2FAmAi2 FUi2FCi2FAmAi2FGmAmUmUm UmGmC*mU*mAGalNAc(L96) | 7 | mU*i2FA*mGi2FCmAi2FAmAmU mCmUmUmGmAi2FUmUi2FUmU mGmCmGmA*mC*mU | 8 |
| mA*mG*i2FCmCmAmAi2FAmAi 2FUi2FCi2FAmAmGmAmUmUm UmGmCmUmAGalNAc(L96) | 9 | mU*i2FA*mGmCmAi2FAmAmUm Ci2FUmUmGmAi2FUmUi2FUmUm GmGmCmU*mC*mU | 10 |
| mU*mG*i2FCmCmAmAi2FAmAi 2FUi2FCi2FAmAmGmAmUmUm UmGmCmUmAGalNAc(L96) | 11 | mU*i2FA*mGmCmAi2FAmAmUm Ci2FUmUmGmAi2FUmUi2FUmUm GmGmCmA*mC*mU | 12 |
| mA*mC*i2FCmCmAmAi2FAmAi 2FUi2FCi2FAmAmGmAmUmUm UmGmCmUmAGalNAc(L96) | 13 | mU*i2FA*mGmCmAi2FAmAmUm Ci2FUmUmGmAi2FUmUi2FUmUm GmGmGmU*mC*mU | 14 |
| mA*mG*i2FGmCmAmAi2FAmAi 2FUi2FCi2FAmAmGmAmUmUm UmGmCmUmAGalNAc(L96) | 15 | mU*i2FA*mGmCmAi2FAmAmUm Ci2FUmUmGmAi2FUmUi2FUmUm GmCmCmU*mC*mU | 16 |
| mC*mA*mCmCmUmUi2FUmUi2 FCi2FUi2FUmCi2FUmAmAmUm GmAmGmUmU | 17 | mA*i2FA*mCmUmCi2FAmUmUm AmGmAmAmGi2FAmAi2FAmAm GmGmUmG*i2FG*mG | 18 |
| mG*mA*mCmCmUmUi2FUmUi2 FCi2FUi2FUmCi2FUmAmAmUm GmAmGmUmU | 19 | mA*i2FA*mCmUmCi2FAmUmUm AmGmAmAmGi2FAmAi2FAmAm GmGmUmC*i2FG*mG | 20 |
| mC*mU*mCmCmUmUi2FUmUi2 FCi2FUi2FUmCi2FUmAmAmUm GmAmGmUmU | 21 | mA*i2FA*mCmUmCi2FAmUmUm AmGmAmAmGi2FAmAi2FAmAm GmGmAmG*i2FG*mG | 22 |
| mC*mA*mGmCmUmUi2FUmUi2 FCi2FUi2FUmCi2FUmAmAmUm GmAmGmUmU | 23 | mA*i2FA*mCmUmCi2FAmUmUm AmGmAmAmGi2FAmAi2FAmAm GmCmUmG*i2FG*mG | 24 |
| mU*mC*mCmCmAmCi2FCmUi2 FUi2FUi2FUmCi2FUmUmCmUi2 FAmAmUmGmAGalNAc(L96) | 25 | mU*i2FC*mAmUmUi2FAmGmAm AmGmAmAmAi2FAmGi2FGmUm GmGmGmA*i2FG*mA | 26 |
| mA*mC*mCmCmAmCi2FCmUi2 FUi2FUi2FUmCi2FUmUmCmUi2 FAmAmUmGmAGalNAc(L96) | 27 | mU*i2FC*mAmUmUi2FAmGmAm AmGmAmAmAi2FAmGi2FGmUm GmGmGmU*i2FG*mA | 28 |
| mU*mG*mCmCmAmCi2FCmUi2 FUi2FUi2FUmCi2FUmUmCmUi2 FAmAmUmGmAGalNAc(L96) | 29 | mU*i2FC*mAmUmUi2FAmGmAm AmGmAmAmAi2FAmGi2FGmUm GmGmCmA*i2FG*mA | 30 |
| mU*mC*mGmCmAmCi2FCmUi2 FUi2FUi2FUmCi2FUmUmCmUi2 FAmAmUmGmAGalNAc(L96) | 31 | mU*i2FC*mAmUmUi2FAmGmAm AmGmAmAmAi2FAmGi2FGmUm GmCmGmA*i2FG*mA | 32 |
| mC*mC*mCmCmAmCfAmAfUfG fAmUfGmAmUmUmAmAmAmC mA | 33 | (*E*)-VP-mU*fG*mUmUmUfAmAm UmCmAmUmCmAfUmUfGmUmG mGmGmG*fG*mG | 34 |
| mG*mC*mCmCmAmCfAmAfUfG fAmUfGmAmUmUmAmAmAmC mA | 35 | (*E*)-VP-mU*fG*mUmUmUfAmAm UmCmAmUmCmAfUmUfGmUmG mGmGmC*fG*mG | 36 |
| mC*mG*mCmCmAmCfAmAfUfG fAmUfGmAmUmUmA mAmAmCmA | 37 | (*E*)-VP-mU*fG*mUmUmUfAmAm UmCmAmUmCmAfUmUfGmUmG mGmCmG*fG*mG | 38 |
| mC*mC*mGmCmAmCfAmAfUfG fAmUfGmAmUmUmAmAmAmC mA | 39 | (*E*)-VP-mU*fG*mUmUmUfAmAm UmCmAmUmCmAfUmUfGmUmG mCmGmG*fG*mG | 40 |
| mC*mA*mCmCmUmUi2FUmUi2 FCi2FUi2FUmCi2FUmAmAmUm GmAmGmUmAGalNAc(L96) | 41 | (*E*)-VPmU*i2FA*mCmUmCi2FAm UmUmAmGmAmAmGi2FAmAi2F AmAmGmGmUmG*i2FG*mG | 42 |
| mC*mU*mCmCmUmUi2FUmUi2 FCi2FUi2FUmCi2FUmAmAmUm | 43 | (*E*)-VPmU*i2FA*mCmUmCi2FAm UmUmAmGmAmAmGi2FAmAi2F | 44 |
| GmAmGmUmAGalNAc(L96) | | AmAmGmGmAmG*i2FG*mG | |
| mC*mA*mCmCmUmUi2FUmUi2 FCi2FUi2FUmCi2FUmAmAmUm GmAmGmUmAGalNAc(L96) | 41 | (E)-VPmU*i2FA*mCmUmCi2FAi2F UmUmAmGmAmAmGi2FAmAi2F AmAmGmGmUmG*i2FG*mG | 45 |
| mC*mU*mCmCmUmUi2FUmUi2 FCi2FUi2FUmCi2FUmAmAmUm GmAmGmUmAGalNAc(L96) | 43 | (*E*)-VPmU*i2FA*mCmUmCi2FAi2F UmUmAmGmAmAmGi2FAmAi2F AmAmGmGmAmG*i2FG*mG | 46 |

### [Examples]

Hereinafter, in order to facilitate the understanding of the present disclosure, exemplary embodiments will be provided. However, the following examples are provided only for easier understanding of the present disclosure, and are not intended to limit the present disclosure.

Unless otherwise noted, the materials and reagents used in the examples are commercially available. The nucleic acid electrophoresis, real-time PCR, and other operations used were performed according to the conventional protocol. This can be done, for example, as described by Molecular Cloning (Cold Spring Harbor Laboratory Press (1989)).

### Example 1: siRNA synthesis

Both the gene and siRNA used in this example were obtained by general solid-phase synthesis methods. The specific synthesis method was as follows.

### 1.1 Methods of synthesis

For the sense strand and antisense strand of the siRNA duplexes and the sense strand and antisense strand of the modified duplexes of the present disclosure, the sense strand was synthesized using standard CPG as the solid support, and the antisense strand was synthesized using universal CPG.

Sequence synthesis was performed using a multi-channel synthesizer. The phosphoramidite monomer was used at a concentration of 0.05-0.1 M, and the activator was 0.3 M BTT (5-benzylthiotetrazole)/1.0 M DCI (4,5-dicyanoimidazole)/0.25-0.6 M ETT (5-ethylthiotetrazole). For siRNA with a ligand coupled to the 3' end of the sense strand, the ligand was introduced by condensation and click after ligation of the targeting ligand to a long-chain amino CPG vector, followed by chain extension according to standard phosphoramidite methods or synthesis of the 3' hexylamine sequence using a 3'-PT Amino-Modifier C6 CPG.

### 1.2 Cleavage and deprotection methods

Cleavage and deprotection of the sequence were performed in 1.5-10 mL tubes. For the naked sequence, AMA (equal volume ratio of ammonia solution to methylamine in ethanol) was used in the first step, and triethylamine trihydrofluoride was used in the second step to deprotect the 2' position. For sequences containing all modifications at the 2' position, ammonolysis with ammonia solution or AMA was used. With Acetone: sequences after cleavage and deprotection were precipitated with a mixture of ethanol (80:20 by volume) or methanol and solubilized with RNA enzyme-free water. Sequences were analyzed by LC-MS to determine sequence accuracy, quantified by a spectrophotometer, and purity was determined by HPLC.

### 1.3 Recovery and separation method

Following HPLC purification, lyophilization, and quality control, the substance was subjected to either sodium acetate/ethanol precipitation or buffer exchange with physiological saline. Subsequent desalting was achieved using a 3 KD molecular weight cutoff ultrafiltration unit. The desalted single strands were quantified by spectrophotometry to determine the concentrations of the sense strand and antisense strand. The sense strand and antisense strand were then mixed at a 1:1 molar ratio in 1x PBS and annealed to form siRNA duplexes (including the modified duplexes or conjugates listed in Table 1).

As an example, the conjugation of the targeting ligand L96 to the duplex could be carried out based on the method disclosed in Example 1 of the description of the patent CN106574268B.

### Example 2: In vitro activity assay

### In vitro activity of the ANGPTL3 gene

### Cell culture and transfection

Cell culture: Hep3B cells (ATCC) were cultured to near confluence in MEM complete medium (Gibco, supplemented with 10% FBS) at 37°C, 5% CO₂, then plated by trypsinization, 2.0 x 10⁵ Hep3B cells and 1.0 mL MEM complete medium (Gibco, supplemented with 10% FBS) were added to each well of 12-well plates, and transfected after 16-24 h at 37°C, 5% CO₂.

Cell Transfection: the two were mixed at a ratio of 1.5 µL lipofectamine RNAiMax (Invitrogen) per 48.5 µL opti-MEM medium, then 50 µL of opt-MEM dilution of each siRNA test sample (modified concatemer or conjugate) prepared by the synthetic method of Example 1 was added to the mixture, the resulting mixture was added to a PCR tube and incubated for 5 min at room temperature, and finally the siRNA transfection complex was added to the cells in the above 12-well plate, followed by RNA extraction after further culture for 24 h. Single-dose experiments were performed at 100 nM, 10 nM, or 1 nM and 0.1 nM duplex or conjugate concentrations.

RNA extraction: Total RNA isolation kit (VAZYME Inc. Cat: RC112-01) was used. The test was performed according to the instructions of the RNA isolation kit. Finally, 50 µL of RNA enzyme-free water was added, and after standing for 5 min, centrifugation was performed at 2,000 rpm (13,400 xg) for 1 min to elute the RNA. cDNA synthesis: cDNA synthesis kit (VAZYME, Cat: RC333-01) was removed by using gDNA for cDNA synthesis. 400 ng of total RNA was added to each sample, and cDNA synthesis was performed using a gradient thermocycler (Bio-rad, T100) according to the manufacturer's protocol.

Quantitative real-time PCR: the synthesized cDNA and master mix (containing primers, qPCR premix, and ultrapure water) were added to a 96-well plate (Thermofisher, Cat: A36924) to establish the final quantitative real-time PCR system, which included either the target gene (ANGPTL3) or the reference gene (GADPH) with forward and reverse primers at 0.4 µM each, and 1x SYBR Green premix (Thermofisher, Cat: A25742).

Real-time fluorescent PCR was performed using the ΔΔCt assay in the ABI QuantStudio 1 Plus real-time fluorescent PCR system. Each duplex was tested in 2-3 independent transfections, in triplicate for each transfection.

Two exemplary groups of conjugates for detection are shown below:

### Group 1:

The sense strand and antisense strand of the first group of conjugates, G1-1, were as follows, wherein the antisense strand and the target gene were mismatch-free:
Sense strand: 
5’-mA*mG*mCmCmAmAi2FAmAi2FUi2FCi2FAmAi2FGmAmUmUmUmGmC*m
U*mAGalNAc(L96)-3’ (SEQ ID NO: 1);
Antisense strand:

5’-mU*i2FA*mGi2FCmAi2FAmAmUmCmUmUmGmAi2FUmUi2FUmUmGmGmC
mU*mC*mU-3’ (SEQ ID NO: 2);

The sense strand and antisense strand of the first group of conjugates G1-2 were as follows, wherein the nucleotide at position 21 of the antisense strand in the 5' end to 3' end direction was mismatched to the target gene:
Sense strand: 
5’-mU*mG*mCmCmAmAi2FAmAi2FUi2FCi2FAmAi2FGmAmUmUmUmGmC*m
U*mAGalNAc(L96)-3’ (SEQ ID NO: 3);
Antisense strand:

5’-mU*i2FA*mGi2FCmAi2FAmAmUmCmUmUmGmAi2FUmUi2FUmUmGmGmC
mA*mC*mU-3’ (SEQ ID NO: 4);

The sense strand and antisense strand of the first group of conjugates G1-3 were as follows, wherein the nucleotides at positions 20 and 21 of the antisense strand in the 5' end to 3' end direction were mismatched to the target gene:
Sense strand: 
5’-mU*mC*mCmCmAmAi2FAmAi2FUi2FCi2FAmAi2FGmAmUmUmUmGmC*m
U*mAGalNAc(L96)-3’ (SEQ ID NO: 5);
Antisense strand:

5’-mU*i2FA*mGi2FCmAi2FAmAmUmCmUmUmGmAi2FUmUi2FUmUmGmGm
GmA*mC*mU-3’ (SEQ ID NO: 6);

The sense strand and antisense strand of the first group of conjugates G1-4 were as follows, wherein the nucleotides at positions 19 to 21 of the antisense strand in the 5' end to 3' end direction were mismatched to the target gene:
Sense strand: 
5’-mU*mC*mGmCmAmAi2FAmAi2FUi2FCi2FAmAi2FGmAmUmUmUmGmC*m
U*mAGalNAc(L96)-3’ (SEQ ID NO: 7);
Antisense strand:

5’-mU*i2FA*mGi2FCmAi2FAmAmUmCmUmUmGmAi2FUmUi2FUmUmGmCmG
mA*mC*mU-3’ (SEQ ID NO: 8).

### Group 2:

The sense strand and antisense strand of the second group of conjugates G2-1 were as follows, with no mismatches between the antisense strand and the target gene:
Sense strand: 
5'-mA*mG*i2FCmCmAmAi2FAmAi2FUi2FCi2FAmAmGmAmUmUmUmGmCmU
mAGalNAc(L96)-3' (SEQ ID NO: 9),
Antisense strand:

5’-mU*i2FA*mGmCmAi2FAmAmUmCi2FUmUmGmAi2FUmUi2FUmUmGmGmC
mU*mC*mU-3’ (SEQ ID NO: 10);

The sense strand and antisense strand of the second group of conjugates G2-2 were as follows, wherein the nucleotide at position 21 of the antisense strand in the 5' end to 3' end direction was mismatched to the target gene:
Sense strand: 
5’-mU*mG*i2FCmCmAmAi2FAmAi2FUi2FCi2FAmAmGmAmUmUmUmGmCmU
mAGalNAc(L96)-3’ (SEQ ID NO: 11),
Antisense strand:

5'-mU*i2FA*mGmCmAi2FAmAmUmCi2FUmUmGmAi2FUmUi2FUmUmGmGmC
mA*mC*mU-3' (SEQ ID NO: 12);

The sense strand and antisense strand of the second group of conjugates G2-3 were as follows, wherein nucleotides at position 20 and 21 of the antisense strand in the 5' end to 3' end direction were mismatched to the target gene:
Sense strand: 
5’-mA*mC*i2FCmCmAmAi2FAmAi2FUi2FCi2FAmAmGmAmUmUmUmGmCmU
mAGalNAc(L96)-3’ (SEQ ID NO: 13),
Antisense strand:

5’-mU*i2FA*mGmCmAi2FAmAmUmCi2FUmUmGmAi2FUmUi2FUmUmGmGm
GmU*mC*mU-3’ (SEQ ID NO: 14);

The sense strand and antisense strand of the second group of conjugates G2-4 were as follows, wherein nucleotides at position 19 to 21 of the antisense strand in the 5' end to 3' end direction were mismatched to the target gene:
Sense strand: 
5’-mA*mG*i2FGmCmAmAi2FAmAi2FUi2FCi2FAmAmGmAmUmUmUmGmCmU
mAGalNAc(L96)-3’ (SEQ ID NO: 15),
Antisense strand:

5’-mU*i2FA*mGmCmAi2FAmAmUmCi2FUmUmGmAi2FUmUi2FUmUmGmCmC
mU*mC*mU-3’ (SEQ ID NO: 16).

The positive control PC A was selected from the AD05488 in Patent Application WO2019055633A1, the antisense strand was AM07235-AS, and the sense strand was AM07234-SS.

The results of the in vitro activity test of the exemplary two groups of conjugates described above are shown in Tables 2 and 3.

**Table 2 Single dose test results for the first group of conjugates (mean ± SEM)**

| Conjugates | 100 nM siRNA | | 10 nM siRNA | |
|---|---|---|---|---|
| | Mean | SEM | Mean | SEM |
| G1-1 | 0.418 | 0.034 | 0.480 | 0.057 |
| G1-2 | 0.210 | 0.028 | 0.367 | 0.048 |
| G1-3 | 0.416 | 0.106 | 0.428 | 0.130 |
| G1-4 | 0.399 | 0.059 | 0.930 | 0.019 |
| PC A | 0.436 | 0.099 | 0.446 | 0.088 |

**Table 3 Single dose test results for the second group of conjugates (mean ± SEM)**

| Conjugates | 1 nM siRNA | | 0.1 nM siRNA | |
|---|---|---|---|---|
| | Mean | SEM | Mean | SEM |
| G2-1 | 0.424 | 0.036 | 0.606 | 0.131 |
| G2-2 | 0.271 | 0.006 | 0.606 | 0.073 |
| G2-3 | 0.289 | 0.051 | 0.638 | 0.023 |
| G2-4 | 0.276 | 0.090 | 0.923 | 0.023 |
| PC A | 0.146 | 0.036 | 0.188 | 0.049 |

### (II) In vitro activity of the AGT gene

### Cell culture and transfection

Cell culture: Hep3B cells were cultured to near confluency in DMEM complete medium (Gibco supplemented with 10% FBS and 1% Penicillin-Streptomycin) at 37°C, 5% CO₂, trypsinized, plated, and transfected after 16-24 h of culture at 37°C, 5% CO₂ with 1.0 x 10⁵ Hep3B cells and 1.0 mL DMEM complete medium per well in 24-well plates.

Cell Transfection: the two were mixed at a ratio of 3 µL lipofectamineRNAiMax (Invitrogen) per 47 µL opti-MEM medium, then 50 µL of each siRNA test sample (modified concatemer or conjugate) prepared by the synthetic method of Example 1 was added to the opt-MEM dilution, the resulting mixture was added to a centrifuge tube and incubated at room temperature for 5 min, and finally the siRNA transfection complex was added to the above cells, followed by RNA extraction after further culture for 24 h. Single-dose experiments were performed at 1 nM and 0.1 nM siRNA duplex or conjugate concentrations.

### RNA extraction

Total RNA isolation kit (VAZYME Inc. Cat: RC112-01): perform the test according to the instructions of the RNA isolation kit, and finally add 50 µL of RNA enzyme-free water; after standing for 5 min, 12000 rpm), centrifuge for 1 min to elute the RNA.

### cDNA Synthesis

cDNA synthesis kit (VAZYME, Cat: RC333-01) was removed by using gDNA for cDNA synthesis. 400 ng of total RNA was added to each sample, and cDNA synthesis was performed using a gradient thermocycler (Bio-rad, T100) following the protocol of the instrument instructions for use.

### Quantitative real-time PCR

The synthesized cDNA and master mix (containing primers, qPCR premix, and ultrapure water) were dispensed into a 96-well plate (Thermofisher, Cat: A36924) to achieve the final quantitative real-time PCR reaction system containing: target gene (AGT) or internal reference gene (GAPDH), upstream and downstream primers of 0.4 µM each, 1x SYBR Green premix solution (Thermofisher, Cat: A25742). Real-time fluorescent PCR was performed using the ΔΔCt assay in the ABI QuantStudio 1 Plus real-time fluorescent PCR system. Each duplex was tested in 2-3 independent transfections, in triplicate for each transfection.

RNA extraction: Total RNA isolation kit (VAZYME Inc. Cat: RC112-01) was used. The test was performed according to the instructions of the RNA isolation kit. Finally, 50 µL of RNA enzyme-free water was added, and after standing for 5 min, centrifugation was performed at 2,000 rpm (13,400 xg) for 1 min to elute the RNA. cDNA synthesis: cDNA synthesis kit (VAZYME, Cat: RC333-01) was removed by using gDNA for cDNA synthesis. 400 ng of total RNA was added to each sample, and cDNA synthesis was performed using a gradient thermocycler (Bio-rad, T100) according to the manufacturer's protocol.

Quantitative real-time PCR: the synthesized cDNA and master mix (containing primers, qPCR premix, and ultrapure water) were added to a 96-well plate (Thermofisher, Cat: A36924) to establish the final quantitative real-time PCR system, which included either the target gene (ANGPTL3) or the reference gene (GADPH) with forward and reverse primers at 0.4 µM each, and 1x SYBR Green premix (Thermofisher, Cat: A25742).

Real-time fluorescent PCR was performed using the ΔΔCt assay in the ABI QuantStudio 1 Plus real-time fluorescent PCR system. Each duplex was tested in 2-3 independent transfections, in triplicate for each transfection.

Exemplary modified duplexes and conjugates for detection are shown below:

### Modified duplex 1

Sense strand: 
5’-mC*mA*mCmCmUmUi2FUmUi2FCi2FUi2FUmCi2FUmAmAmUmGmAmGm
UmU-3’ (SEQ ID NO: 17)
Antisense strand:

5'-mA*i2FA*mCmUmCi2FAmUmUmAmGmAmAmGi2FAmAi2FAmAmGmGmU
mG*i2FG*mG-3' (SEQ ID NO: 18);

### Modified duplex 2

Sense strand: 
5’-mG*mA*mCmCmUmUi2FUmUi2FCi2FUi2FUmCi2FUmAmAmUmGmAmGm
UmU-3’ (SEQ ID NO: 19)
Antisense strand:

5'-mA*i2FA*mCmUmCi2FAmUmUmAmGmAmAmGi2FAmAi2FAmAmGmGmU
mC*i2FG*mG-3' (SEQ ID NO: 20);

### Modified duplex 3

Sense strand: 
5’-mC*mU*mCmCmUmUi2FUmUi2FCi2FUi2FUmCi2FUmAmAmUmGmAmGm
UmU-3’ (SEQ ID NO: 21)
Antisense strand:

5’-mA*i2FA*mCmUmCi2FAmUmUmAmGmAmAmGi2FAmAi2FAmAmGmGmA
mG*i2FG*mG-3’ (SEQ ID NO: 22);

### Modified duplex 4

Sense strand: 
5’-mC*mA*mGmCmUmUi2FUmUi2FCi2FUi2FUmCi2FUmAmAmUmGmAmGm
UmU-3’ (SEQ ID NO: 23)
Antisense strand:

5'-mA*i2FA*mCmUmCi2FAmUmUmAmGmAmAmGi2FAmAi2FAmAmGmCmU
mG*i2FG*mG-3' (SEQ ID NO: 24);

### Conjugate 1

Sense strand: 
5'-mU*mC*mCmCmAmCi2FCmUi2FUi2FUi2FUmCi2FUmUmCmUi2FAmAmUm
GmAGalNAc(L96)-3' (SEQ ID NO: 25),
Antisense strand:

5'-mU*i2FC*mAmUmUi2FAmGmAmAmGmAmAmAi2FAmGi2FGmUmGmGmG
mA*i2FG*mA-3' (SEQ ID NO: 26);

### Conjugate 2

Sense strand: 
5'-mA*mC*mCmCmAmCi2FCmUi2FUi2FUi2FUmCi2FUmUmCmUi2FAmAmUm
GmAGalNAc(L96)-3' (SEQ ID NO: 27),
Antisense strand:

5'-mU*i2FC*mAmUmUi2FAmGmAmAmGmAmAmAi2FAmGi2FGmUmGmGmG
mU*i2FG*mA-3' (SEQ ID NO: 28);

### Conjugate 3

Sense strand: 
5’-mU*mG*mCmCmAmCi2FCmUi2FUi2FUi2FUmCi2FUmUmCmUi2FAmAmUm
GmAGalNAc(L96)-3’ (SEQ ID NO: 29),
Antisense strand:

5’-mU*i2FC*mAmUmUi2FAmGmAmAmGmAmAmAi2FAmGi2FGmUmGmGmC
mA*i2FG*mA-3’ (SEQ ID NO: 30);

### Conjugate 4

Sense strand: 
5'-mU*mC*mGmCmAmCi2FCmUi2FUi2FUi2FUmCi2FUmUmCmUi2FAmAmUm
GmAGalNAc(L96)-3' (SEQ ID NO: 31),
Antisense strand:

5’-mU*i2FC*mAmUmUi2FAmGmAmAmGmAmAmAi2FAmGi2FGmUmGmCmG
mA*i2FG*mA-3’ (SEQ ID NO: 32);

The positive reference PC B was:
Sense strand: 
5’-mG*mU*mCmAmUmCi2FCmAi2FCi2FAi2FAmUmGmAmGmAmGmUmAmC
mA-3’ (SEQ ID NO: 47);
Antisense strand:

5'-mU*i2FG*mUmAmCTmCmUmCmAmUmUmGi2FUmGi2FGmAmUmGmAmC
*mG*mA-3' (SEQ ID NO: 48);

The positive reference PC C was:
Sense strand: 
5'-mG*mU*mCmAmUmCi2FCmAi2FCi2FAi2FAmUmGmAmGmAmGmUmAmCm
AGalNAc(L96)-3' (SEQ ID NO: 49);
Antisense strand:

5'-mU*i2FG*mUmAmCTmCmUmCmAmUmUmGi2FUmGi2FGmAmUmGmAmC*
mG*mA-3' (SEQ ID NO: 50)。

The results of the in vitro activity test of exemplary test samples are shown in Tables 4 and 5.

**Table 4 Single-dose test results for modified duplexes**

| Duplex No. | 1 nM siRNA | | 0.1 nM siRNA | |
|---|---|---|---|---|
| | Mean | SEM | Mean | SEM |
| Modified duplex 1 | 0.243 | 0.025 | 0.398 | 0.018 |
| Modified duplex 2 | 0.175 | 0.011 | 0.339 | 0.015 |
| Modified duplex 3 | 0.170 | 0.019 | 0.315 | 0.051 |
| Modified duplex 4 | 0.180 | 0.015 | 0.291 | 0.043 |
| PC B | 0.224 | 0.018 | 0.306 | 0.036 |

**Table 5 Single-dose test results for conjugates**

| Conjugate No. | 1 nM siRNA | | 0.1 nM siRNA | |
|---|---|---|---|---|
| | Mean | SEM | Mean | SEM |
| Conjugate 1 | 0.613 | 0.109 | 0.848 | 0.017 |
| Conjugate 2 | 0.448 | 0.063 | 0.561 | 0.083 |
| Conjugate 3 | 0.320 | 0.008 | 0.558 | 0.041 |
| Conjugate 4 | 0.322 | 0.042 | 0.575 | 0.043 |
| PC C | 0.133 | 0.049 | 0.390 | 0.053 |

### (III) In vitro activity of the RAGE gene

According to the method described in the above section "(II) In vitro activity of AGT gene", the cells were transfected with NCI-H2009 cells (overexpressing Human AGER mRNA; Cyagen Biosciences), in vitro evaluation of dsRNA targeting the RAGE gene, where single dose experiments were performed at 0.01 nM and 0.0001 nM siRNA duplex or conjugate concentrations.

dsRNA duplexes of each group were transfected into a 96-well plate (Thermofisher, Cat. No. A36924) using Lipofectamine RNAiMax transfection reagent (Invitrogen) at final concentrations of 0.01 nM and 0.0001 nM, NCI-H 2009 cells were plated at approximately 2.4 x 10⁴ cells per well, cultured for 16-24 h before RNA extraction, extracted RNA were subjected to cDNA synthesis, then RAGE mRNA expression was compared to an endogenous control by real-time fluorescent quantitative RCR, and relative expression of each group of dsRNA was analysed using the ΔΔCt assay. Each group of dsRNA was tested in 2-3 independent transfections, and three RT-qPCR assays were performed per transfection; the results are shown in Table 6.

Exemplary modified duplexes for detection are shown below:

### Modified Duplex 1

Sense strand: mC*mC*mCmCmAmCfAmAfUfGfAmUfGmAmUmUmAmAmAmCmA (SEQ ID NO: 33),
Antisense strand:

(*E*)-VP-mU*fG*mUmUmUfAmAmUmCmAmUmCmAfUmUfGmUmGmGmGmG*
fG*mG (SEQ ID NO: 34);

### Modified Duplex 2

Sense strand: mG*mC*mCmCmAmCfAmAfUfGfAmUfGmAmUmUmAmAmAmCmA (SEQ ID NO: 35),
Antisense strand:

(*E*)-VP-mU*fG*mUmUmUfAmAmUmCmAmUmCmAfUmUfGmUmGmGmC*f
G*mG (SEQ ID NO: 36);

### Modified Duplex 3

Sense strand: mC*mG*mCmCmAmCfAmAfUfGfAmUfGmAmUmUmA mAmAmCmA (SEQ ID NO: 37),
Antisense strand:

(*E*)-VP-mU*fG*mUmUmUfAmAmUmCmAmUmCmAfUmUfGmUmGmGmCmG*f
G*mG (SEQ ID NO: 38);

### Modified Duplex 4

Sense strand: mC*mC*mGmCmAmCfAmAfUfGfAmUfGmAmUmUmAmAmAmCmA (SEQ ID NO: 39),
Antisense strand:

(*E*)-VP-mU*fG*mUmUmUfAmAmUmCmAmUmCmAfUmUfGmUmGmCmGmG*f
G*mG (SEQ ID NO: 40);

The positive reference substance PC D was:
Sense strand: 
inVAb*mUmAmGmUmAmGmGmUfGfCfUmCmAmAmAmAmCmAmUmCmA*in
VAb (SEQ ID NO: 51),
Antisense strand: (*E*)-VP-mU*fG*mA*fUmGfUmUfUmUfGmAfGmCfAmCfCmUfAmCfU*mA (SEQ ID NO: 52).

**Table 6 Single-dose test results for modified duplexes**

| Duplex No. | 0.01 nM siRNA | | 0.0001 nM siRNA | |
|---|---|---|---|---|
| | Mean | SEM | Mean | SEM |
| PC D | 0.438 | 0.101 | 0.672 | 0.326 |
| Modified duplex 1 | 0.663 | 0.069 | 1.159 | 0.113 |
| Modified duplex 2 | 0.447 | 0.196 | 0.857 | 0.189 |
| Modified duplex 3 | 0.438 | 0.156 | 0.700 | 0.013 |
| Modified duplex 4 | 0.355 | 0.125 | 0.528 | 0.059 |

### Example 3: PHH human hepatic primary cells free uptake

Resuscitation of human primary hepatic cells: 1 mL of human primary liver cells was added into 19 mL of DMEM complete medium, gently inverted and mixed well, centrifuged for 5 min at 50 x g, and the supernatant was discarded. The cell pellet was then resuspended in 10 mL of DMEM complete medium.

Administration: siRNA conjugate diluted with opti-MEM medium was added to a 96-well collagen-coated 96-well cell plate in an amount of 10 µL/well (dosing group), and 5.4 x 10⁴ cells/well of PHH were inoculated into the 96-well cell plate, while a control group containing no siRNA conjugate was set up: an equal volume of nuclease-free water was added. Plates were plated and incubated at 37°C in a 5% CO₂ incubator.

Exemplary conjugates for detection are shown below (PC C is the same as PC C in "(II) In vitro activity of AGT gene" according to Example 2):

### Conjugate 1

Sense strand: 
5’-mC*mA*mCmCmUmUi2FUmUi2FCi2FUi2FUmCi2FUmAmAmUmGmAmGm
UmAGalNAc(L96)-3’ (SEQ ID NO: 41),
Antisense strand:

5'-(E)-VPmU*i2FA*mCmUmCi2FAmUmUmAmGmAmAmGi2FAmAi2FAmAmG
mGmUmG*i2FG*mG-3' (SEQ ID NO: 42);

### Conjugate 2

Sense strand: 
5’-mC*mU*mCmCmUmUi2FUmUi2FCi2FUi2FUmCi2FUmAmAmUmGmAmGm
UmAGalNAc(L96)-3’ (SEQ ID NO: 43),
Antisense strand:

5’-(E)-VPmU*i2FA*mCmUmCi2FAmUmUmAmGmAmAmGi2FAmAi2FAmAmG
mGmAmG*i2FG*mG-3’ (SEQ ID NO: 44);

### Conjugate 3

Sense strand: 
5'-mC*mA*mCmCmUmUi2FUmUi2FCi2FUi2FUmCi2FUmAmAmUmGmAmGm
UmAGalNAc(L96)-3' (SEQ ID NO: 41),
Antisense strand:

5’-(E)-VPmU*i2FA*mCmUmCi2FAi2FUmUmAmGmAmAmGi2FAmAi2FAmAmG
mGmUmG*i2FG*mG-3’ (SEQ ID NO: 45);

### Conjugate 4

Sense strand: 
5’-mC*mU*mCmCmUmUi2FUmUi2FCi2FUi2FUmCi2FUmAmAmUmGmAmGm
UmAGalNAc(L96)-3’ (SEQ ID NO: 43),
Antisense strand:

5'-(E)-VPmU*i2FA*mCmUmCi2FAi2FUmUmAmGmAmAmGi2FAmAi2FAmAmG
mGmAmG*i2FG*mG-3' (SEQ ID NO: 46).

RNA extraction and reverse transcription: after 48 h of free cellular uptake, the medium was removed, and the cells were collected for RNA extraction. Total RNA was extracted using RNeasy^{®} 96 Kit (QIAGEN-74182) according to the kit instructions. Subsequently, following the instructions, the cDNA synthesis kit (VAZYME, Cat: RC333-01) was removed by using gDNA for cDNA synthesis. 400 ng of total RNA was added to each sample, and cDNA synthesis was performed using a gradient thermocycler (Bio-rad, T100) according to the manufacturer's protocol. qPCR to detect the expression level of the target gene mRNA: the synthesized cDNA and master mix (containing primers, 1× SYBR Green premix, and ultrapure water) were added into a 96-well plate (Thermofisher, Cat: A36924). The final quantitative real-time PCR reaction system contained the following components: target gene (AGT) or internal reference gene (GADPH), upstream and downstream primers of 0.4 µM each, and 1x SYBR Green premix solution (Thermofisher, Cat: A25742).

Data Analysis: according to the CT value of each sample, the expression level of target gene AGT mRNA in the sample was calculate, and a calculation was made by the ΔΔCT relative quantitative method. The relative expression of the target gene was expressed using 2-ΔΔCT.

The calculation formula is as follows: $ΔCT = mean CT value of target gene - mean CT value of internal reference gene$ $ΔΔCT = ΔCT \left(dosing group\right) - ΔCT \left(nuclease-free water control group\right)$ $Relative expression of target gene mRNA = {2}^{- ΔΔCT}$ 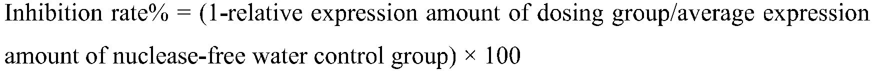 Inhibition rate% = (1-relative expression amount of dosing group/average expression amount of nuclease-free water control group) × 100

The in vitro activity test results of the conjugates are shown in Table 7, and the test results are expressed as mean ± SD.

**Table 7 Human liver primary cell-free uptake test results for siRNA conjugates**

| Duplex No. | 10 nM siRNA | | 1 nM siRNA | |
|---|---|---|---|---|
| | Mean | SD | Mean | SD |
| PC C | 11.10% | 0.390 | 38.6% | 2.814 |
| Conjugate 1 | 10.59% | 0.304 | 39.4% | 2.503 |
| Conjugate 2 | 9.23% | 0.339 | 30.2% | 4.727 |
| Conjugate 3 | 11.75% | 0.870 | 39.8% | 0.474 |
| Conjugate 4 | 9.55% | 0.415 | 26.9% | 4.507 |

### Example 4: In vivo activity assay-in vivo silencing of AGT in transgenic mice expressing human AGT

Using transgenic mice expressing human AGT, mice were given a single subcutaneous dose of 1 mg/kg of siRNA conjugate (3 mice/group; Conjugate 3, Conjugate 4, and PC C in Example 3), along with the PBS control group (3/group). Blood was collected 1 day prior to administration and at 7-day intervals after dosing. After the blood was centrifuged to extract serum, the expression level of hAGT protein was detected using the ELISA method, and KD was calculated using the expression amount of hAGT protein 1 day before administration as a baseline value. The results of the in vivo activity test in mice of the conjugates to be tested are shown in Table 8, and the test results were expressed as mean ± SD.

**Table 8 Test results of in vivo mouse assay**

| Conjugate No. | Time | KD means of serum AGT | SD |
|---|---|---|---|
| Conjugate 3 (1mg/kg, n=3) | -1 day | 1.00 | 0.03 |
| | 7 days | 0.17 | 0.01 |
| | 14 days | 0.22 | 0.01 |
| Conjugate 4 (1mg/kg, n=3) | -1 day | 1.00 | 0.09 |
| | 7 days | 0.13 | 0.01 |
| | 14 days | 0.15 | 0.03 |
| PC C (1mg/kg, n=3) | -1 day | 1.00 | 0.03 |
| | 7 days | 0.45 | 0.10 |
| | 14 days | 0.38 | 0.06 |
| PBS (n=3) | -1 day | 1.00 | 0.16 |
| | 7 days | 0.90 | 0.14 |
| | 14 days | 0.84 | 0.12 |

While particular embodiments of the present disclosure have been described above, it will be understood by those skilled in the art that these are merely illustrative and that various changes or modifications may be made to these embodiments without departing from the principles and spirit of the present disclosure. Accordingly, the protection sought herein is as set forth in the claims below.

## Claims

1. A siRNA for inhibiting gene expression, comprising a sense strand and an antisense strand, with at least one nucleotide at a position greater than 15 of the antisense strand in the 5' end to 3' end direction being mismatched to a target gene.

2. The siRNA according to claim 1, wherein the nucleotide at position 19 of the antisense strand in the 5' end to 3' end direction is mismatched to the target gene.

3. The siRNA according to claim 1, wherein the nucleotide at position 20 of the antisense strand in the 5' end to 3' end direction is mismatched to the target gene.

4. The siRNA according to claim 1, wherein the nucleotide at position 21 of the antisense strand in the 5' end to 3' end direction is mismatched to the target gene.

5. The siRNA according to any one of claims 1 to 4, wherein the nucleotide is identical to the nucleotide of the target gene at the mismatch position, and the nucleotide of the antisense strand and the nucleotide of the sense strand are complementary paired at the mismatch position.

6. The siRNA according to claim 1, wherein the antisense strand comprises an overhang of 2 nucleotides in length at the 3' end.

7. The siRNA according to claim 1, wherein the number of nucleotides of the sense strand is 21 and the number of nucleotides of the antisense strand is 23.

8. The siRNA according to claim 1, wherein all nucleotides are modified nucleotides.
